(19) Europäisches Patentamt
European Patent Office
Office européen des brevets

(11) Veröffentlichungsnummer: **0 224 078 A1**

# (12) EUROPÄISCHE PATENTANMELDUNG

(21) Anmeldenummer: 86115204.9

(22) Anmeldetag: 03.11.86

(51) Int. Cl.4: C07D 409/12 , A01N 47/44

(30) Priorität: 18.11.85 DE 3540840

(43) Veröffentlichungstag der Anmeldung: 03.06.87 Patentblatt 87/23

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Fest, Christa, Dr.
Im Johannistal 20
D-5600 Wuppertal 1(DE)
Erfinder: Kirsten, Rolf, Dr.
Carl-Langhans-Strasse 27
D-4019 Monheim(DE)
Erfinder: Kluth, Joachim, Dr.
Kurt-Schumacher-Strasse 9
D-4018 Langenfeld(DE)
Erfinder: Müller, Klaus-Helmut, Dr.
Bockhackstrasse 55
D-4000 Düsseldorf 13(DE)
Erfinder: Pfister, Theodor, Dr.
Lichtenberger Strasse 30
D-4019 Monheim(DE)
Erfinder: Priesnitz, Uwe, Dr.
Severinstrasse 58
D-5650 Solingen(DE)
Erfinder: Riebel, Hans-Jochem, Dr.
In Der Beek 92
D-5600 Wuppertal 1(DE)
Erfinder: Roy, Wolfgang, Dr.
Walter-Kolb-Strasse 47
D-4018 Langenfeld(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch-Gladbach 2(DE)

(54) **Thienylsulfonylguanidin-Derivate.**

(57) Die Erfindung betrifft neue Thienylsulfonylguanidin-Derivate der allgemeinen Formel (I)

EP 0 224 078 A1

Xerox Copy Centre

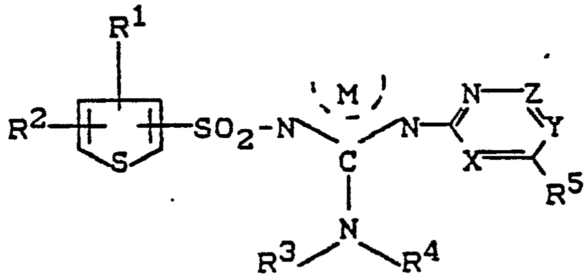

(I)

(worin die Reste M, $R^1$, $R^2$, $R^3$, $R^4$, $R^5$, X, Y und Z die in der Beschreibung angegebenen Bedeutungen haben) sowie 1 : 1-Addukte von Verbindungen der Formel (I) mit starken Säuren, Verfahren zu ihrer Herstellung und ihre Verwendung als Herbizide.

## Thienylsulfonylguanidin-Derivate

Die Erfindung betrifft neue Thienylsulfonylguanidin-Derivate, Verfahren zu deren Herstellung und deren Verwendung als Herbizide.

Es ist bekannt, daß bestimmte Guanidine, wie z. B. N'-(4,6-Dimethyl-pyrimidin-2-yl)-N''-hydroxy-N'''-(2-chlor-benzolsulfonyl)-guanidin, herbizid wirksam sind. Die Wirkung dieser Verbindungen ist jedoch nicht immer ganz befriedigend (vergl. EP-OS 117 014).

Es wurden nun neue Thienylsulfonylguanidin-Derivate der allgemeinen Formel (I)

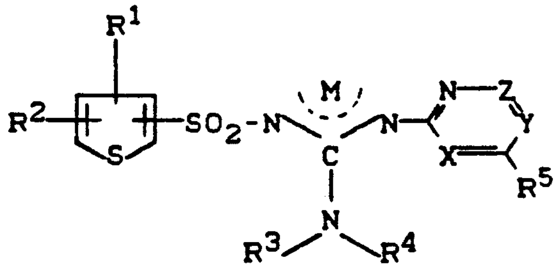

(I)

in welcher

$R^1$ und $R^2$ gleich oder verschieden sind und für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxy-carbonyl [welches gegebenenfalls durch Fluor, Chlor und/oder $C_1$-$C_4$-Alkoxy substituiert ist] stehen,

$R^3$ für Wasserstoff, $C_1$-$C_4$-Alkyl oder für einen Sulfonylrest der Formel $R^6$-$SO_2$-steht, worin

$R^6$ für den Rest

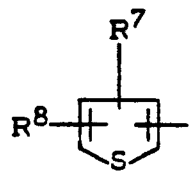

steht, worin

$R^7$ und $R^8$ gleich oder verschieden sind und für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl) – aminosulfonyl oder $C_1$-$C_4$-Alkoxy-carbonyl [welches gegebenenfalls durch Fluor, Chlor und/oder $C_1$-$C_4$-Alkoxy substituiert ist] stehen,

in welcher weiter

$R^6$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-$C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxycarbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [wel ches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_2$-$C_{12}$-Alkenyloxy [welches gegebenenfalls durch Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, $C_3$-$C_6$-Cycloalkylamino, Di-($C_3$-$C_6$-cycloalkyl)-amino oder Di-( $C_1$-$C_4$-alkyl)-amino steht [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino (welches gegebenenfalls durch Fluor, Chlor, Brom, Cyano, Nitro oder Phenyl substituiert ist], für Phenylamino oder Benzylamino -[welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl

substituiert sind], für Benzyl oder Phenyl steht, wobei letztere gegebenenfalls durch einen oder mehrere Reste aus der Reihe Halogen, Cyano, Nitro, $C_1$-$C_4$-Alkyl [welches gegebenenfalls durch Fluor, Chlor, Brom, Cyano, Carboxy, $C_1$-$C_4$-Alkoxy-carbonyl, $C_1$-$C_4$-Alkyl-amino-carbonyl, $C_1$-$C_4$-Alkoxy-amino-carbonyl, Di-( $C_1$-$C_4$-alkyl)-amino-carbonyl, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkyl-amino-carbonyloxy, $C_1$-$C_4$-Alkylthio, $C_1$-$C_4$-Alkylsulfinyl, $C_1$-$C_4$-Alkylsulfonyl, Di-( $C_1$-$C_4$-alkyl)-amino-sulfonyl oder Phenyl substituiert ist], $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], $C_2$-$C_6$-Alkinyl [welches gegebenenfalls durch Fluor, Chlor, Brom, Cyano, $C_1$-$C_4$-Alkoxy-carbonyl oder Phenyl substituiert ist], $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkenylthio [welches gegebenenfalls durch Fluor, Chlor, Brom, Cyano oder $C_1$-$C_4$-Alkoxy-carbonyl substituiert ist], $C_1$-$C_4$-Alkylthio, $C_1$-$C_4$-Alkylsulfinyl oder $C_1$-$C_4$-Alkylsulfonyl [welche gegebenenfalls durch Fluor, Chlor, Brom, Cyano oder $C_1$-$C_4$-Alkoxy-carbonyl substituiert sind], Phenyl, Phenoxy, Di-($C_1$-$C_4$-alkyl)-amino-sulfonyl, $C_1$-$C_4$-Alkyl-amino-sulfonyl, $C_1$-$C_4$-Alkoxy-$C_1$-$C_4$-alkyl-amino-sulfonyl, $C_1$-$C_4$-Alkoxy-sulfonyl, $C_1$-$C_4$-Alkoxy-amino-sulfonyl, $C_1$-$C_6$-Alkoxy-carbonyl, $C_3$-$C_6$-Cycloalkoxy-carbonyl, $C_1$-$C_6$-Alkylamino-carbonyl, Di-($C_1$-$C_4$-alkyl)-amino-carbonyl, $C_1$-$C_6$-Alkoxy-amino-carbonyl, $C_1$-$C_4$-Alkoxy-$C_1$-$C_4$-alkyl-amino-carbonyl, $C_1$-$C_6$-Alkyl-aminosulfonylamino oder Di-($C_1$-$C_6$-alkyl)-amino-sulfonylamino substituiert sind und/oder gegebenenfalls benzanelliert sind;

in welcher weiter

$R^4$ für Wasserstoff, $C_1$-$C_6$-Alkyl [welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht, oder

$R^3$ und $R^4$ gemeinsam für $C_4$-$C_6$-Alkandiyl stehen [welches gegebenenfalls durch eine Sauerstoff-Brücke oder durch eine Brücke >N-$R^9$ unterbrochen ist], wobei

$R^9$ für $C_1$-$C_4$-Alkyl, $C_1$-$C_4$-Alkyl-carbonyl, $C_1$-$C_4$-Alkoxy-carbonyl oder Phenyl steht [welches gegebenenfalls durch Fluor, Chlor, Brom, Cyano, Nitro, $C_1$-$C_4$-Alkyl, $C_1$-$C_2$-Fluoralkyl oder $C_1$-$C_4$-Alkoxy substituiert ist];

in welcher weiter

$R^4$ für den Rest -O$R^{10}$ steht, worin

$R^{10}$ für $C_1$-$C_{12}$-Alkyl [welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist], $C_3$-$C_6$-Alkenyl [welches gegebenenfalls durch Fluor oder Chlor substituiert ist], $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-amino-carbonyl-$C_1$-$C_2$-alkyl, Di-($C_1$-$C_4$-alkyl)-amino-carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxy-carbonyl substituiert sind] steht und

in welcher weiter

$R^4$ für den Rest

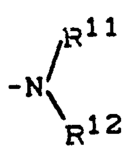

steht, worin

$R^{11}$ für Wasserstoff oder $C_1$-$C_4$-Alkyl steht und

$R^{12}$ für Wasserstoff, $C_1$-$C_4$-Alkyl [welches gegebenenfalls durch Halogen, Cyano, Nitro, $C_1$-$C_4$-Alkoxy oder $C_1$-$C_4$-Alkoxy-carbonyl substituiert ist], $C_3$-$C_6$-Cycloalkyl, Phenyl-$C_1$-$C_2$-alkyl, Phenyl [welche gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiert sind], für $C_1$-$C_4$-Alkyl-carbonyl, $C_1$-$C_4$-Alkoxy-carbonyl, $C_1$-$C_4$-Alkylsulfonyl, $C_1$-$C_4$-Halogenalkylsulfonyl oder Phenylsulfonyl [welches gegebenenfalls durch Halogen, Cyano, Nitro, $C_1$-$C_4$-Alkyl, $C_1$-$C_4$-Halogenalkyl, $C_1$-$C_4$-Alkoxy, $C_1$-$C_2$-Halogenalkoxy oder $C_1$-$C_4$-Alkoxy-carbonyl substituiert ist] steht,

$R^5$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy,

$C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,
X für Stickstoff oder eine -CH-Gruppierung steht,
Y für Stickstoff oder eine -$CR^{13}$-Gruppierung steht, worin
$R^{13}$ für Wasserstoff, Halogen, $C_1$-$C_4$-Alkyl, Formyl, $C_1$-$C_4$-Alkyl-carbonyl, $C_1$-$C_4$-Alkoxy-carbonyl oder $C_1$-$C_2$-Alkoximino-($C_1$-$C_2$-alkyl) steht, und
Z für Stickstoff oder eine -$CR^{14}$-Guppierung steht, worin
$R^{14}$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)-amino steht und
M für Wasserstoff oder ein Metalläquivalent steht,
sowie 1:1-Addukte von Verbindungen der Formel (I) mit starken Säuren gefunden.

Die allgemeine Formel (I) steht -wenn M für Wasserstoff steht -für die einzelnen Tautomeren der Formeln (IA) und (IB)

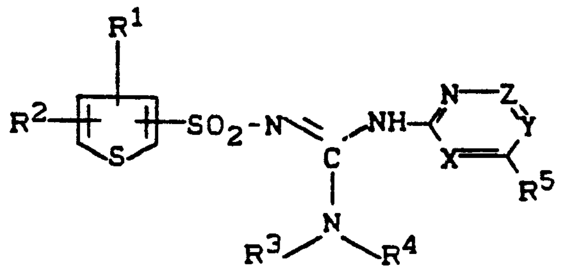

(IA)

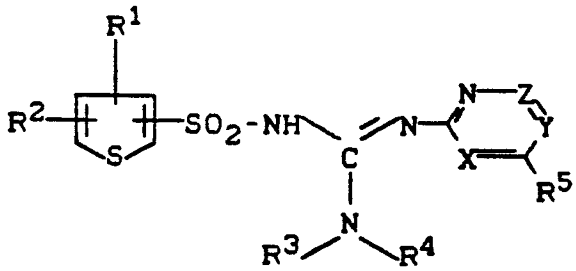

(IB)

in welchen
$R^1$, $R^2$, $R^3$, $R^4$, $R^5$, X, Y und Z die oben angegebene Bedeutung haben,
sowie für Gemische der Tautomeren (IA) und (IB).

Das Mischungsverhältnis (IA)/(IB) hängt von aggregationsbestimmenden Faktoren, wie z. B. Temperatur, Lösungsmittel und Konzentration ab.

Für den Fall, daß neben M auch $R^3$ für Wasserstoff steht, ist eine weitere tautomere Form (IC) möglich:

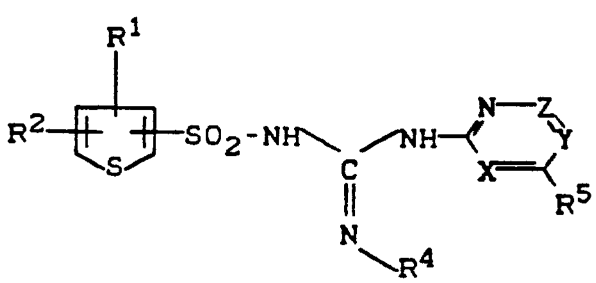

(IC)

Man erhält die neuen Thienylsulfonylguanidin-Derivate der Formel (I)
(a) für den Fall, daß M für Wasserstoff steht,
wenn man Guanidin-Derivate der Formel (II)

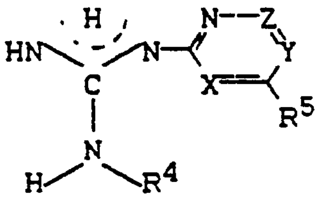

(II)

in welcher
$R^4$, $R^5$, X, Y und Z die oben angegebenen Bedeutungen haben,
mit Sulfonsäurechloriden der Formel (III)

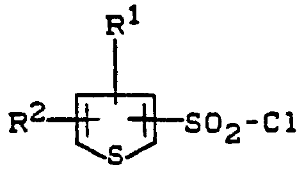

(III)

in welcher
$R^1$ und $R^2$ die oben angegebenen Bedeutungen haben,
in Gegenwart von Säureakzeptoren und gegebenenfalls in Gegenwart von Verdünnungsmitteln umsetzt;
oder

(b) für den Fall, daß M für Wasserstoff steht und $R^3$ für Wasserstoff oder $C_1$-$C_4$-Alkyl steht,
wenn man die nach dem oben unter (a) angegebenen Verfahren erhältlichen Thienylsulfonylguanidin-Derivate der Formel (ID)

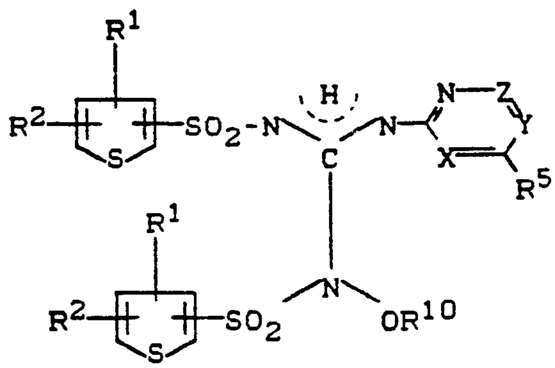

(ID)

in welcher
$R^1$, $R^2$, $R^5$, $R^{10}$, X, Y und Z die oben angegebenen Bedeutungen haben,
mit Aminoverbindungen der Formel (IV)

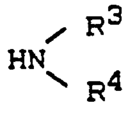

(IV)

in welcher
$R^3$ für Wasserstoff oder $C_1$-$C_4$-Alkyl steht und
$R^4$ die oben angegebene Bedeutung hat,
bzw. mit Hydrochloriden von Aminoverbindungen der Formel (IV), gegebenenfalls in Gegenwart von Säure akzeptoren und gegebenenfalls in Gegenwart von Verdünnungsmitteln umsetzt;

oder

(c) für den Fall, daß M für Wasserstoff steht und $R^3$ für einen Sulfonylrest der Formel $R^6-SO_2$-steht, worin $R^6$ die oben angegebene Bedeutung hat, wenn man Thienylsulfonylguanidin-Derivate der Formel (IE)

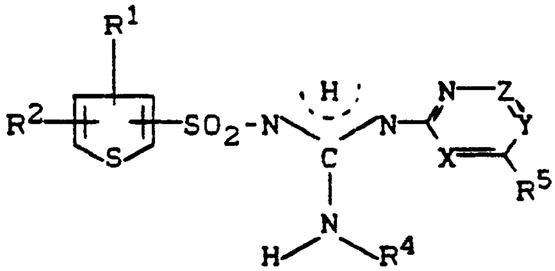

(IE)

in welcher $R^1$, $R^2$, $R^4$, $R^5$, X, Y und Z die oben angegebenen Bedeutungen haben, mit Sulfonsäurechloriden der Formel (V)

$R^6-SO_2-Cl$ (V)

in welcher $R^6$ die oben angegebene Bedeutung hat, in Gegenwart von Säureakzeptoren und gegebenenfalls in Gegenwart von Verdünnungsmitteln umsetzt; oder

(d) ebenfalls für den Fall, daß M für Wasserstoff steht und $R^3$ für einen Sulfonylrest der Formel $R^6-SO_2$-steht, worin $R^6$ die oben angegebene Bedeutung hat, wenn man Sulfonylguanidin-Derivate der Formel (VI)

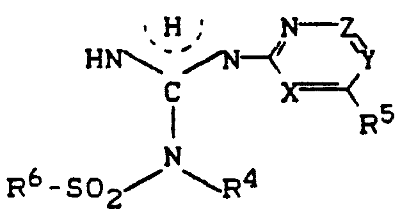

(VI)

in welcher $R^4$, $R^5$, $R^6$, X, Y und Z die oben angegebenen Bedeutungen haben, mit Sulfonsäurechloriden der Formel (III)

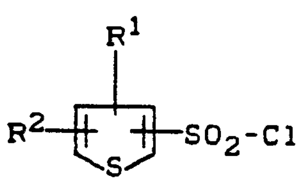

(III)

in welcher $R^1$ und $R^2$ die oben angegebenen Bedeutungen haben, in Gegenwart von Säureakzeptoren und gegebenenfalls in Gegenwart von Verdünnungsmitteln umsetzt; oder

(e) für den Fall, daß M für Wasserstoff steht und $R^3$ für Wasserstoff oder $C_1$-$C_4$-Alkyl steht, wenn man Sulfonylisothioharnstoff-Derivate der Formel (VII)

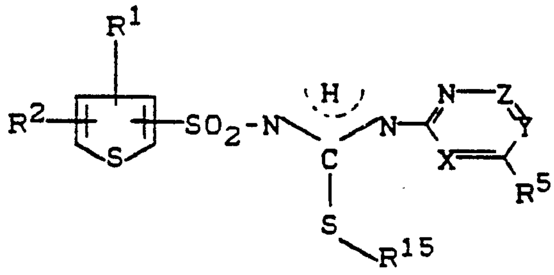

(VII)

in welcher
$R^1$, $R^2$, $R^5$, X, Y und Z die oben angegebenen Bedeutungen haben und
$R^{15}$ für $C_1$-$C_4$-Alkyl oder Benzyl steht,
mit Aminoverbindungen der Formel (IV)

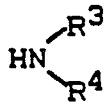

(IV)

in welcher
$R^3$ für Wasserstoff oder $C_1$-$C_4$-Alkyl steht und
$R^4$ die oben angegebene Bedeutung hat,
gegebenenfalls in Gegenwart von Verdünnungsmitteln umsetzt;
oder

(f) für den Fall, daß M für ein Metalläquivalent steht, wenn man die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$, $R^5$, X, Y und Z die oben angegebenen Bedeutungen haben,
mit Metallhydroxiden, -hydriden oder -alkanolaten oder mit metallorganischen Verbindungen, gegebenenfalls in Gegenwart von Verdünnungsmitteln umsetzt:
oder

(g) für den Fall, daß 1:1-Addukte von Verbindungen der Formel (I) mit starken Säuren herzustellen sind,
wenn man Verbindungen der Formel (I), in welcher M für Wasserstoff steht und $R^1$, $R^2$, $R^3$, $R^4$, $R^5$, X, Y und Z die oben angegebenen Bedeutungen haben,
mit starken Säuren, gegebenenfalls in Gegenwart von Verdünnungsmitteln umsetzt.

Die neuen Thienylsulfonylguanidin-Derivate der Formel (I) und ihre Addukte mit starken Säuren zeichnen sich durch eine starke herbizide Wirksamkeit aus.

Überraschenderweise zeigen die neuen Verbindungen der Formel (I) erheblich bessere herbizide Wirkung als vorbekannte Guanidine gleicher Wirkungsrichtung.

Gegenstand der Erfindung sind vorzugsweise Verbindungen der Formel (I), in welcher
M für Wasserstoff oder ein Natrium-, Kalium-oder Calcium-äquivalent steht,
$R^1$ für Wasserstoff, Chlor, Methyl, Cyano, Nitro oder Methoxy steht,
$R^2$ für Wasserstoff, Chlor, Methyl, $C_1$-$C_3$-Alkoxy, $C_1$-$C_3$-Alkylthio, $C_1$-$C_3$-Alkylsulfinyl, $C_1$-$C_3$-Alkylsulfonyl, Dimethylaminosulfonyl oder $C_1$-$C_3$-Alkoxycarbonyl steht,
$R^3$ für Wasserstoff, Methyl oder für einen Sulfonylrest der Formel $R^6$-$SO_2$-steht, worin
$R^6$ für den Rest

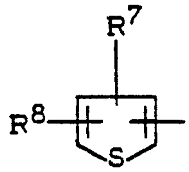

steht, worin

$R^7$ für Wasserstoff, Chlor, Methyl, Cyano, Nitro oder Methoxy steht,

$R^8$ für Wasserstoff, Chlor, Methyl, $C_1$-$C_3$-Alkoxy, $C_1$-$C_3$-Alkylthio, $C_1$-$C_3$-Alkylsulfinyl, $C_1$-$C_3$-Alkylsulfonyl, Dimethylaminosulfonyl oder $C_1$-$C_3$-Alkoxycarbonyl steht,

in welcher weiter

$R^6$ für $C_1$-$C_4$-Alkyl [welches gegebenenfalls durch Fluor und/oder Chlor substituiert ist], für $C_1$-$C_4$-Alkylamino [welches gegebenenfalls durch Fluor und/oder Chlor substituiert ist], für Di-($C_1$-$C_2$-alkyl)-amino, für Benzyl oder Phenyl [welche gegebenenfalls durch Fluor, Chlor, Brom, Cyano, Nitro, $C_1$-$C_4$-Alkyl, Trifluormethyl, $C_1$-$C_4$-Alkoxy, Trifluormethoxy, Difluormethoxy, $C_1$-$C_4$-Alkylthio, $C_1$-$C_4$-Alkoxycarbonyl, $C_1$-$C_4$-Alkylsulfinyl, $C_1$-$C_4$-Alkylsulfonyl, Dimethylaminosulfonyl, Phenyl oder Phenoxy substituiert sind], steht

in welcher weiter

$R^4$ für den Rest -O-$R^{10}$ steht, worin

$R^{10}$ für $C_1$-$C_8$-Alkyl [welches gegebenenfalls durch Fluor oder Chlor substituiert ist], für $C_3$-$C_6$-Alkenyl - [welches gegebenenfalls durch Chlor substituiert ist], für $C_1$-$C_3$-Alkoxy-carbonyl-$C_1$-$C_2$-alkyl, Phenyl, Phenylethyl oder Benzyl [welches gegebenenfalls durch Fluor, Chlor, Nitro, Methyl, Methoxy oder $C_1$-$C_2$-Alkoxy-carbonyl substituiert ist] steht;

in welcher weiter

$R^4$ für den Rest

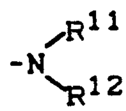

steht, worin

$R^{11}$ für Wasserstoff oder Methyl steht und

$R^{12}$ für Wasserstoff, $C_1$-$C_3$-Alkyl [welches gegebenenfalls durch Fluor, Chlor, Cyano, $C_1$-$C_2$-Alkoxy oder $C_1$-$C_2$-Alkoxy-carbonyl substituiert ist], für $C_3$-$C_6$-Cycloalkyl, Phenyl, Benzyl oder Phenylethyl [welche gegebenenfalls durch Fluor, Chlor, Brom, Nitro, Cyano, $C_1$-$C_2$-Alkyl, $C_1$-$C_2$-Alkoxy oder $C_1$-$C_2$-Alkoxy-carbonyl substituiert sind], für Acetyl, Methoxycarbonyl, Benzolsulfonyl oder Toluolsulfonyl steht;

in welcher weiter

$R^5$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,

X für Stickstoff oder eine -CH-Gruppierung steht,

Y für Stickstoff oder eine -$CR^{13}$-Gruppierung steht, worin

$R^{13}$ für Wasserstoff, Fluor, Chlor, Brom, Methyl, Formyl, Acetyl, Methoxycarbonyl, Ethoxycarbonyl oder 1-(1-Methoximino)-ethyl steht,

und

Z für Stickstoff oder eine -$CR^{14}$-Gruppierung steht, worin

$R^{14}$ für Wasserstoff, Fluor, Chlor, Brom, Methyl, Ethyl, Methoxy, Ethoxy, Propoxy, Isopropoxy, Methylthio, Ethylthio, Methylamino, Ethylamino,Dimethylamino oder Diethylamino steht.

Gegenstand der Erfindung sind vorzugsweise ferner 1:1-Addukte von Verbindungen der Formel (I) -wie vorausgehend definiert, wobei M für Wasserstoff steht -mit Halogenwasserstoffsäuren, wie Hydrogenchlorid, Hydrogenbromid und Hydrogeniodid, mit Schwefelsäure, Trifluoressigsäure, mit gegebenenfalls durch Fluor oder Chlor substituierten Alkansulfonsäuren mit bis zu 4 Kohlenstoffatomen oder auch mit Benzol-oder Naphthalin-sulfonsäuren, welche gegebenenfalls durch Fluor, Chlor, Brom oder Methyl substituiert sind.

Verwendet man beispielsweise für die Verfahrensvariante (a) 2-Ethoxycarbonyl-3-thienylsulfonsäurechlorid und N'-(4-Methoxy-6-methyl-pyrimidin-2-yl)-N''-ethoxy-guanidin als Ausgangsstoffe, so kann der Reaktionsablauf durch folgendes Formelschema skizziert werden:

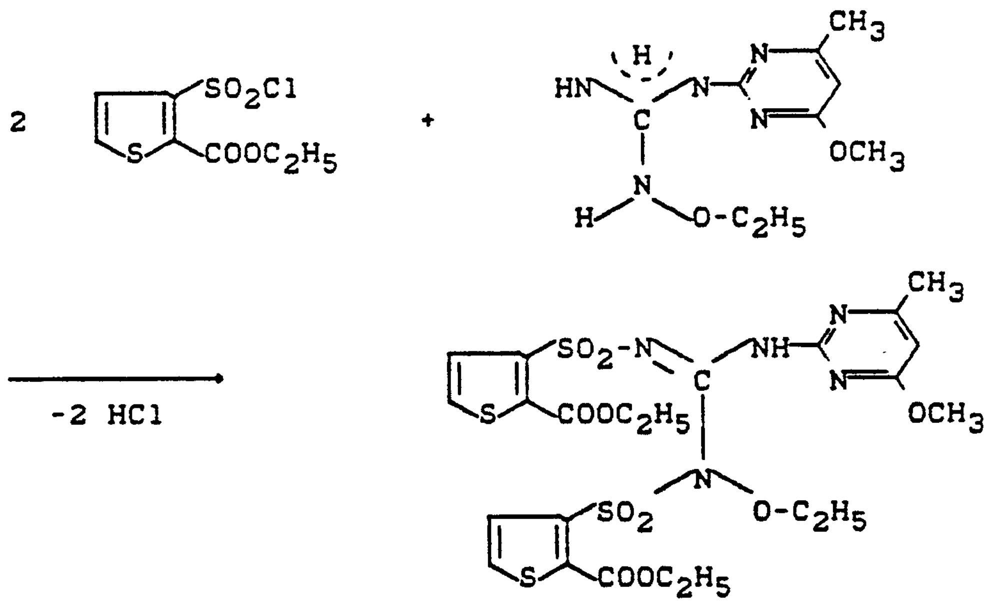


Verwendet man beispielsweise für die Verfahrensvariante (b) N'-(4-Methyl-pyrimidin-2-yl)-N''-methoxy-N'',N'''-bis-(2-methoxycarbonyl-3-thienylsulfonyl)-guanidin und Phenylhydrazin als Ausgangsstoffe, so kann der Reaktionsablauf durch das folgende Formelschema skizziert werden:

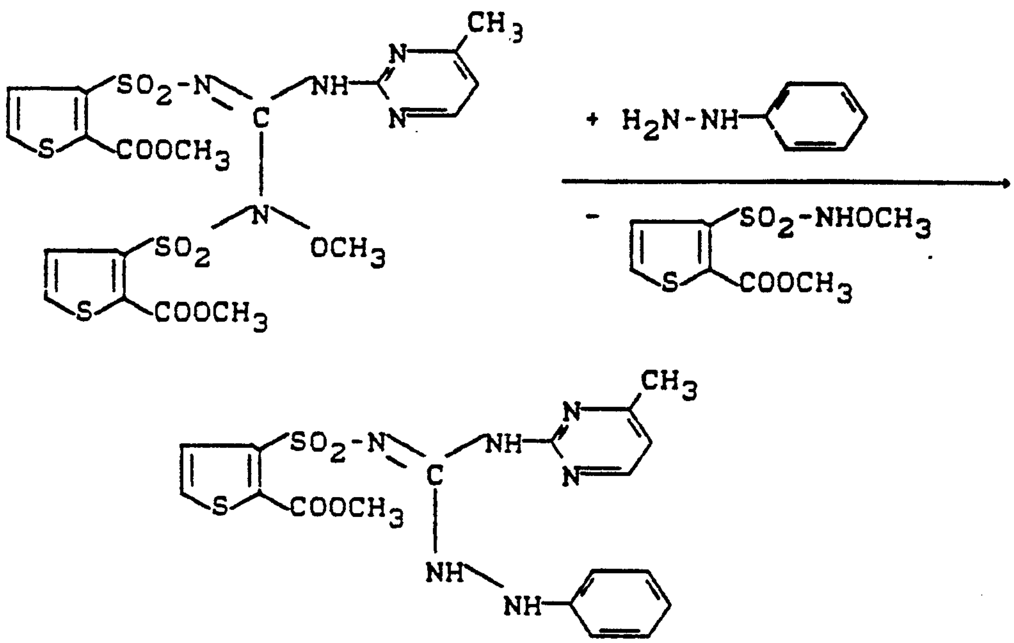

Verwendet man beispielsweise für die Verfahrensvariante (c) N'-(4,6-Dimethoxy-1,3,5-triazin-2-yl)-N''-benzyloxy-N'''-(2-methoxycarbonyl-3-thienylsulfonyl)-guanidin und Trifluormethan-sulfonsäurechlorid als Ausgangsstoffe, so kann der Reaktionsablauf durch das folgende Formelschema skizziert werden:

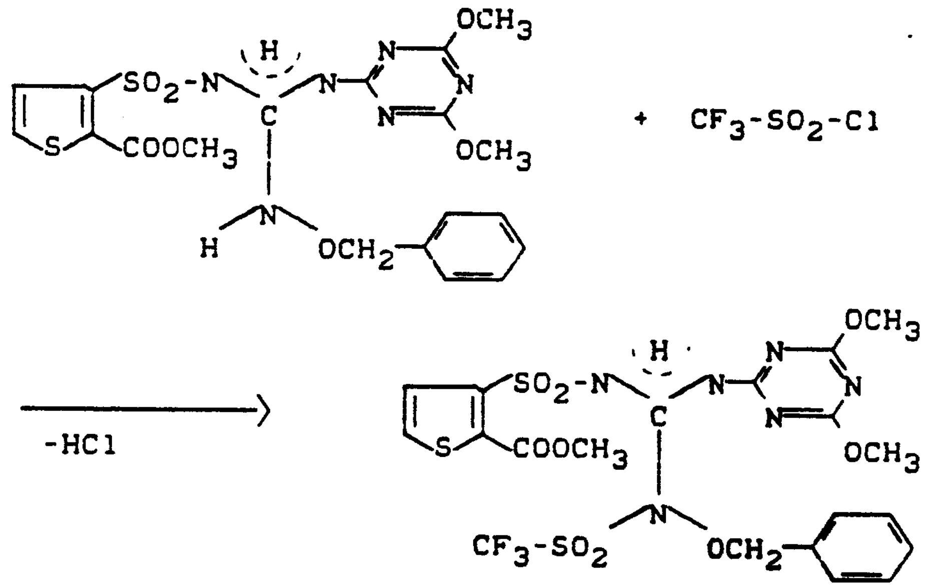


Verwendet man beispielsweise für die Verfahrensvariante (d) N'-(4-Ethoxy-6-methyl-pyrimidin-2-yl)-N"-methoxy-N"-(2-ethoxycarbonyl-benzolsulfonyl)-guanidin und 2-Chlor-3-thienylsulfonsäurechlorid als Ausgangsstoffe, so kann der Reaktionsablauf durch folgendes Formelschema skizziert werden:

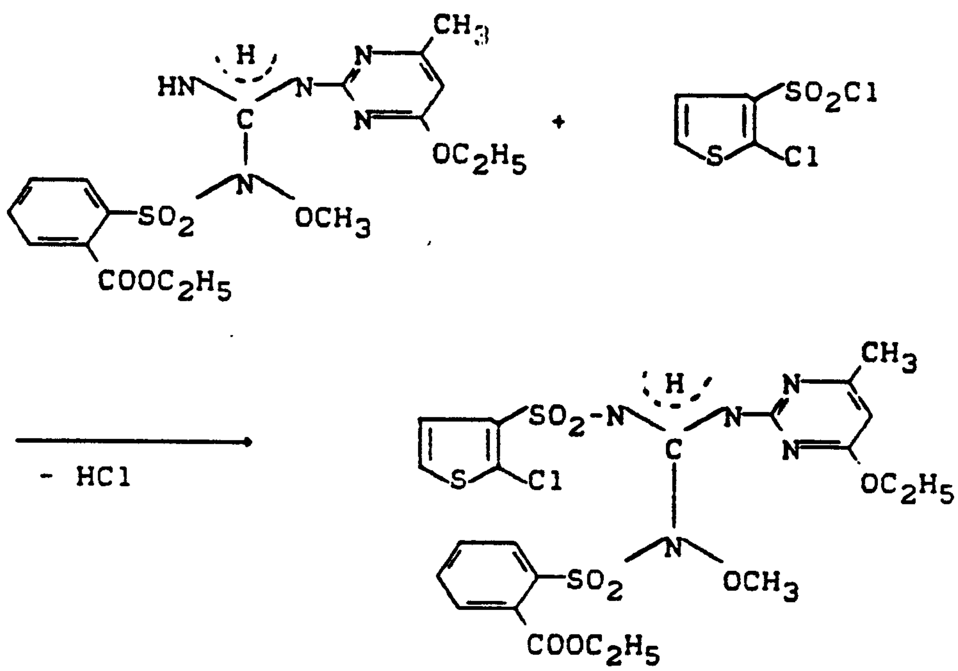


Verwendet man beispielsweise für die Verfahrensvariante (e) N'-(4-Methoxy-6-methyl-1,3,5-triazin-2-yl)-N"-(5-methoxycarbonyl-3-thienylsulfonyl)-S-methyl-isothioharnstoff und N,N-Dimethylhydrazin als Ausgangsstoffe, so kann der Reaktionsablauf durch das folgende Formelschema skizziert werden:

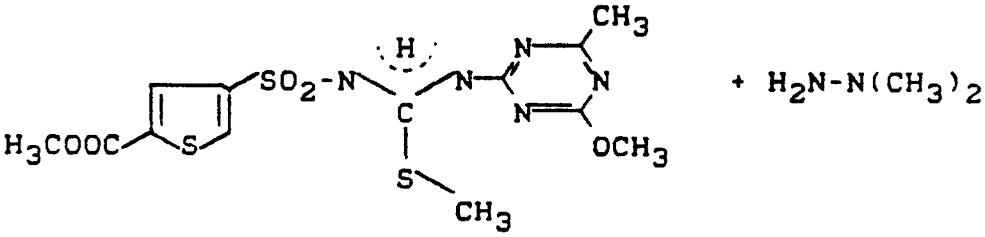

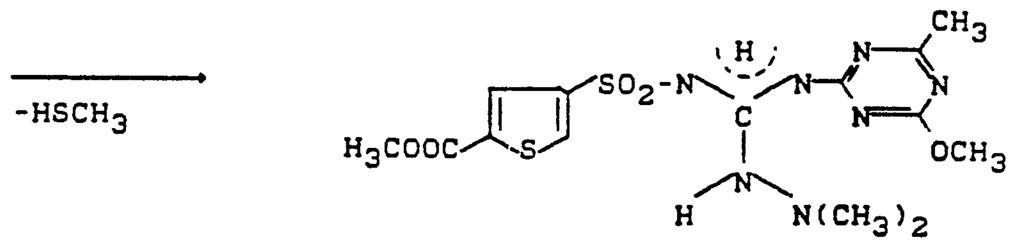


Verwendet man beispielsweise für die Verfahrensvariante (f) N'-(4-Chlor-6-methoxy-pyrimidin-2-yl)-N''-isopropoxy-N'''-(3-thienylsulfonyl)-guanidin und Kaliumethanolat als Ausgangsstoffe, so kann der Reaktionsablauf durch das folgende Formelschema skizziert werden:

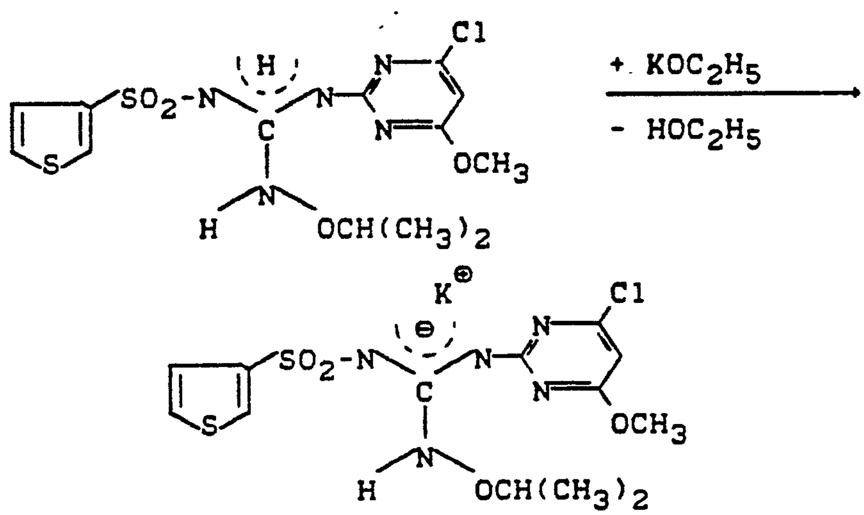


Verwendet man beispielsweise für die Verfahrensvariante (g) N'-(4,6-Dimethyl-1,3,5-triazin-2-yl)-N''-methoxy-N''-(2-chlor-benzolsulfonyl)-N'''-(5-chlor-2-thienylsulfonyl)-guanidin und Methansulfonsäure als Ausgangsstoffe, so kann der Reaktionsablauf durch folgendes Formelschema skizziert werden:

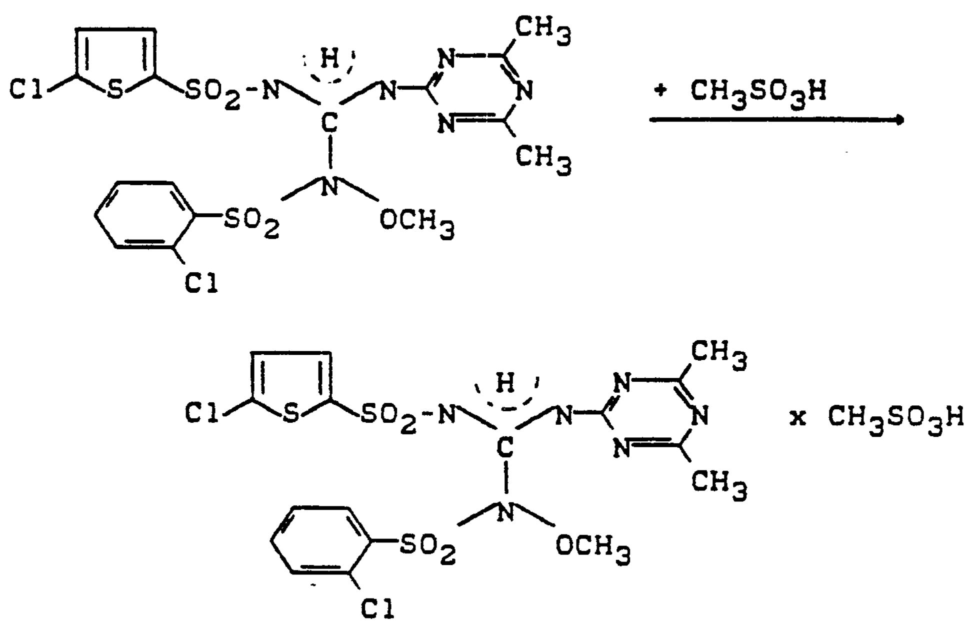

Die als Ausgangsstoffe für die Verfahrensvariante (a) zu verwendenden Guanidin-Derivate sind durch die Formel (II) allgemein definiert. In Formel (II) haben $R^4$, $R^5$, X, Y und Z vorzugsweise die gleichen Bedeutungen, wie sie oben im Rahmen der Substituentendefinition der Formel (I) vorzugsweise angegeben sind.

Als Ausgangsstoffe der Formel (II) seien beispielsweise genannt:

N'-(4-Methyl-pyrimidin-2-yl)-, N'-(4,6-Dimethyl-pyrimidin-2-yl)-, N'-(4-Ethyl-pyrimidin-2-yl)-, N'-(4-Methoxy-6-methyl-pyrimidin-2-yl)-, N'-(4-Ethoxy-6-methyl-pyrimidin-2-yl)-, N'-(4-Propoxy-6-methyl-pyrimidin-2-yl)-, N'-(4-Isopropoxy-6-methyl-pyrimidin-2-yl)-, N'-(4-Chlor-6-methoxy-pyrimidin-2-yl)-, N'-(4-Chlor-6-ethoxy-pyrimidin-2-yl)-, N'-(4-Chlor-6-dimethylamino-pyrimidin-2-yl)-, N'-(4-Methyl-6-methylthio-pyrimidin-2-yl)-, N'-(4,6-Dimethoxy-pyrimidin-2-yl)-, N'-(4-Difluormethoxy-6-methyl-pyrimidin-2-yl)-und N'-(4-Dimethylamino-6-methyl-pyrimidin-2-yl)-N''-methoxy-guanidin, -N''-ethoxy-guanidin, -N''-propoxy-guanidin, -N''-isopropoxy-guanidin, -N''-butoxy-guanidin, -N''-isobutoxy-guanidin, -N''-sec.-butoxy-guanidin, -N''-pentoxy-guanidin, -N''-isopentoxy-guanidin, -N''-hexyloxy-guanidin, -N''-octyloxy-guanidin, -N''-allyloxy-guanidin, -N''-(2-chlor-ethoxy-)-guanidin, -N''-(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy-)-guanidin, -N''-methoxycarbonylmethoxy-guanidin, -N''-(ethoxy-carbonyl-methoxy)-guanidin, -N''-(1-methoxycarbonyl-ethoxy)-guanidin, -N''-(1-ethoxycarbonyl-ethoxy)-guanidin, -N''-(dimethylaminocarbonyl-methoxy)-guanidin, -N''-(2-phenyl-ethoxy)-guanidin, -N''-phenoxy-guanidin, -N''-(4-methyl-benzyloxy)-guanidin, -N''-(4-fluor-benzyloxy)-guanidin, -N''-(4-chlor-benzyloxy)-guanidin, -N''-(4-nitro-benzyloxy)-guanidin, -N''-(2,6-dichlorbenzyloxy)-guanidin, -N''-(4-methoxycarbonyl-benzyloxy)-guanidin und -N''-(4-ethoxycarbonyl-benzyloxy)-guanidin; N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-4,6-(Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-und N'-(4-Diethylamino-6-ethylthio-s-triazin-2-yl)-N''-methoxy-guanidin, -N''-ethoxy-guanidin, -N''-propoxy-guanidin, -N''-isopropoxy-guanidin, -N''-butoxy-guanidin, -N''-isobutoxy-guanidin, -N''-sec.-bu toxy-guanidin, -N''-pentoxy-guanidin, -N''-isopentoxy-guanidin, -N''-hexyloxy-guanidin, -N''-octyloxy-guanidin, -N''-allyloxy-guanidin, -N''-(2-chlor-ethoxy)-guanidin, -N''-(2-fluor-ethoxy)-guanidin, -N''-(2-chlor-propoxy)-guanidin, -N''-(2-fluor-propoxy)-guanidin, -N''-(3-chlor-propoxy)-guanidin, -N''-(4-chlor-butoxy)-guanidin, -N''-methoxycarbonylmethoxy-guanidin, -N''-ethoxycarbonylmethoxy-guanidin, -N''-(1-methoxy-carbonylethoxy)-guanidin, -N''-(1-ethoxycarbonyl-ethoxy)-guanidin, -N''-dimethylamino-carbonylmethoxy-guanidin, -N''-(2-phenyl-ethoxy)-guanidin, -N''-phenoxy-guanidin, -N''-(4-methylbenzyloxy)-guanidin, -N''-(4-fluor-benzyloxy)-guanidin, -N''-(4-chlor-benzyloxy)-guanidin, -N''-(4-nitro-benzyloxy)-guanidin, -N''-(2,6-dichlor-benzyloxy)-guanidin, -N''-(4-methoxycarbonylbenzyloxy)-guanidin und -N''-(4-ethoxycarbonyl-benzyloxy)-guanidin.

Die Ausgangsstoffe der Formel (II) sind bekannt (vgl. DE-OS 3 334 455).

Die weiter als Ausgangsstoffe für die Verfahrensvariante (a) zu verwendenden Sulfonsäurechloride sind durch die Formel (III) allgemein definiert. In Formel (III) haben $R^1$ und $R^2$ vorzugsweise die gleichen Bedeutungen, wie sie oben im Rahmen der Substituentendefinition der Formel (I) vorzugsweise genannt sind.

Als Ausgangsstoffe der Formel (III) seien beispielsweise genannt:

2-Chlor-3-thienyl-, 3-Chlor-2-thienyl-, 2-Methoxycarbonyl-3-thienyl-, 2,5-Dichlor-3-thienyl-, 2-Thienyl-, 3-Thienyl-, 5-Chlor-2-thienyl-, 5-Methoxycarbonyl-3-thienyl-, 5-Methoxycarbonyl-2-thienyl-, 2-Ethoxycarbonyl-3-thienyl-sulfonsäurechlorid und 3-Methoxycarbonyl-2-thienyl-sulfonsäurechlorid.

Die Sulfonsäurechloride der Formel (III) sind bekannt und/oder können nach an sich bekannten Verfahren hergestellt werden (vergl. z. B. US-PS 4 127 405, US-PS 4 169 719, US-PS 4 481 029, US-PS 4 441 910, EP-A 17 473 und Herstellungsbeispiel).

Die als Ausgangsstoffe für die Verfahrensvariante (b) zu verwendenden Thienylsulfonylguanidin-Derivate sind durch die Formel (ID) allgemein definiert. In Formel (ID) haben $R^1$, $R^2$, $R^5$, $R^{10}$, X, Y und Z vorzugsweise die gleichen Bedeutungen, wie sie oben im Rahmen der Substituentendefinition der Formel (I) vorzugsweise angegeben sind.

Als Beispiele für die Verbindungen der Formel (ID) seien genannt:
N'-(4-Chlor-6-methoxy-pyrimidin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und -N'',N'''-bis-(5-methoxycarbonyl-2-thienylsulfonyl)-guanidin;
N'-(4,6-Dimethyl-pyrimidin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Methyl-pyrimidin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4,6-Dimethoxy-pyrimidin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Methoxy-6-methyl-pyrimidin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Difluormethoxy-6-methyl-pyrimidin-2-yl)-N''-methoxy-
-N'',N'''-bis-(2-chlor-3-thienylsulfonyl)-,
-N'',N'''-bis-(3-chlor-2-thienylsulfonyl)-,

-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4,6-Dimethyl-s-triazin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Methoxy-6-methyl-s-triazin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4,6-Dimethoxy-s-triazin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Ethyl-6-methoxy-s-triazin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(4-Dimethylamino-6-methyl-s-triazin-2-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(5,6-Dimethyl-1,2,4-triazin-3-yl)-N''-methoxy-

-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N-(5-Methyl-1,2,4-triazin-3-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin;
N'-(2,6-Dimethyl-pyrimidin-4-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin und
N'-(2,6-Dimethoxy-pyrimidin-4-yl)-N''-methoxy-
-N'',N'''-bis-(2-chlor-3-thienylsulfonyl)-,
-N'',N'''-bis-(3-chlor-2-thienylsulfonyl)-,
-N'',N'''-bis-(2-methoxycarbonyl-3-thienylsulfonyl)-,
-N'',N'''-bis-(2,5-dichlor-3-thienylsulfonyl)-,
-N'',N'''-bis-(2-thienylsulfonyl)-,
-N'',N'''-bis-(3-thienylsulfonyl)-,
-N'',N'''-bis-(5-chlor-2-thienylsulfonyl)-,
-N'',N'''-bis-(5-methoxycarbonyl-3-thienylsulfonyl)-und
-N'',N'''-bis-(5-methoxycarbonyl-2-thienylsulfonyl)-guanidin.

Die Verbindungen der Formel (ID) können nach dem oben unter (a) beschriebenen Verfahren hergestellt werden.

Die bei der Verfahrensvariante (b) weiter als Ausgangstoffe zu verwendenden Aminoverbindungen sind durch die Formel (IV) allgemein definiert. In Formel (IV) haben $R^3$ und $R^4$ vorzugsweise die gleichen Bedeutungen, wie sie oben im Rahmen der Substituentendefinition der Formel (I) vorzugsweise angegeben worden sind.

Als Ausgangsstoffe der Formel (IV) seien beispielsweise genannt:
N,N-Dimethylhydrazin, Phenylhydrazin, O-Methyl-hydroxylamin, O-Ethyl-hydroxylamin, O,N-Dimethylhydroxylamin, O-Propyl-, O-Isopropyl-, O-Butyl-, O-sec.-Butyl-, O-Pentyl-, O-Isopentyl-, O-sec.-Pentyl-, O-Hexyl-, O-Isohexyl-, O-Heptyl-, O-Isoheptyl-, O-Octyl-, O-Isooctyl-, O-Allyl, O-Crotyl-, O-(2-Chlor-ethyl)-, O-(2-Fluor-ethyl)-, O-(2-Chlor-propyl)-, O-(3-Chlor-propyl)-, O-(4-Chlor-butyl)-, O-Methoxycarbonylmethyl-, O-Ethoxycarbonylmethyl-, O-(1-Methoxycarbonyl)-ethyl-, O-(1-Ethoxycarbonyl)-ethyl-, O-Aminocarbonylmethyl-, O-(2-Phenyl-ethyl)-, O-Phenyl-, O-(4-Methyl-benzyl)-, O-(4-Fluor-benzyl)-, O-(4-Chlor-benzyl)-, O-(4-Nitro-benzyl)-, O-(2,6-Dichlor-benzyl)-, O-(4-Methoxycarbonyl-benzyl)-und O-(4-Ethoxycarbonyl-benzyl)-hydroxylamin.

Aminoverbindungen der Formel (IV) sind bekannt und können nach an sich bekannten Verfahren hergestellt werden (vergl. Chem. Pharm. Bull. 15 , (1967), 345; Bull. Soc. Chim. France 1958, 664; Synthesis 1976, 682; J. Chem. Soc. 1930, 228 und Helv. Chim. Acta 45 (1962), 1387).

Die bei der Verfahrensvariante (c) als Ausgangsstoffe zu verwendenden Thienylsulfonylguanidin-Derivate sind durch die Formel (IE) allgemein definiert. In Formel (IE) haben $R^1$, $R^2$, $R^4$, $R^5$, X, Y und Z vorzugsweise die gleichen Bedeutungen, wie sie oben im Rahmen der Substituentendefinition der Formel - (I) vorzugsweise angegeben sind.

Als Beispiele für die Verbindungen der Formel (IE) seien genannt:

-N'-(4,6-Dimethoxy-pyrimidin-2-yl)-N"-methoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4,6-Dimethoxy-pyrimidin-2-yl)-N"-ethoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4,6-Dimethoxy-pyrimidin-2-yl)-N"-propoxy-,
-N"-isopropoxy-,
-N"-butoxy-,
-N"-isobutoxy-,
-N"-sec.-butoxy,-
-N"-pentyloxy-,
-N"-hexyloxy-,
-N"-octyloxy-,
-N"-allyloxy-,
-N"-crotyloxy-,
-N"-(3-chlor-propoxy)-,
-N"-methyloxycarbonyl-methoxy-,
-N"-ethoxycarbonyl-methoxy-,
-N"-(1-methoxy-carbonyl-ethoxy)-,
-N"-(1-ethoxycarbonyl-ethoxy)-,
-N"-(2-phenyl-ethoxy)-,
-N"-phenoxy-,
-N"-benzyloxy-,
-N"-(4-methyl-benzyloxy)-,
-N"-(4-fluor-benzyloxy)-,
-N"-(4-chlor-benzyloxy)-,
-N"-(4-nitro-benzyloxy)-,
-N"-(2,6-dichlor-benzyloxy)-,
-N"-(4-methoxycarbonyl-benzyloxy)-und
-N"-(4-ethoxycarbonyl-benzyloxy)-,
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,

-N'''-(5-chlor-2-thienylsulfonyl)-,
-N'''-(5-methoxycarbonyl-3-thienylsulfonyl)-sowie
-N'''-(5-methoxycarbonyl-2-thienylsulfonyl)--guanidin;
N'-(4-Ethoxy-6-methyl-pyrimidin-2-yl)-und
N'-(4-Methyl-pyrimidin-2-yl)-N''-methoxy-N'''-(2-methoxycarbonyl-3-thienylsulfonyl)-guanidin;
ferner
N'-(4-Chlor-6-methoxy-pyrimidin-2-yl)-N''-methoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
-N'-(4-Chlor-6-ethoxy-pyrimidin-2-yl)-N''-ethoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4-Chlor-6-dimethylamino-pyrimidin-2-yl)-N''-methoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4-Methyl-6-methylthio-pyrimidin-2-yl)-N''-allyloxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4-Dimethylamino-6-methyl-pyrimidin-2-yl)-N''-isopropoxy-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,

-N'''-(5-methoxycarbonyl-3-thienylsulfonyl)-und
-N'''-(5-methoxycarbonyl-2-thienylsulfonyl)-guanidin;
ferner
N'-(4-Difluormethoxy-6-methyl-pyrimidin-2-yl)-N''-methoxycarbonyl-
-N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;
ferner
N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-, N'-(4-Diethylamino-6-ethylthio-s-triazin-2-yl)-,N'-(2,6-Dimethyl-pyrimidin-4-yl)-und N'-(2,6-Dimethoxy-pyrimidin-4-yl)-N''-methoxy-, -N''-ethoxy-, -N''-propoxy-, -N''-isopropoxy-, -N''-butoxy-, -N''-isobutoxy-, -N''-sec.-butoxy, -N''-pentoxy-, -N''-hexyloxy-, -N''octyloxy-, -N''-allyloxy-, -N''-crotyloxy-, -N''-(3-chlor-propoxy)-, -N''-methoxycarbonylmethoxy-, -N''-ethoxycarbonylmethoxy, -N''-(1-methoxycarbonylethoxy)-, -N''-(1-ethoxycarbonylethoxy)-, -N''-(2-phenylethoxy)-, -N''-phenoxy-, -N''-benzyloxy-, -N''-(4-methyl-benzyloxy)-, -N''-(4-fluor-benzyloxy)-, -N''-(4-chlor-benzyloxy)-, -N''-(4-nitro-benzyloxy)-, -N''-(2,6-dichlor-benzyloxy)-, -N''-(4-methoxycarbonylbenzyloxy)-und -N''-(4-ethoxycarbonyl-benzyloxy)-, -N'''-(2-chlor-3-thienylsulfonyl)-,
-N'''-(3-chlor-2-thienylsulfonyl)-,
-N'''-(2-methoxycarbonyl-3-thienylsulfonyl)-,
-N'''-(2,5-dichlor-3-thienylsulfonyl)-,
-N'''-(2-thienylsulfonyl)-,
-N'''-(3-thienylsulfonyl)-,
-N'''-(5-chlor-2-thienylsulfonyl)-,
-N'''-(5-methoxycarbonyl-3-thienylsulfonyl)-und
-N'''-(5-methoxycarbonyl-2-thienylsulfonyl)-guanidin;

Die Verbindungen der Formel (IE) können nach den oben unter (a) und (b) beschriebenen Verfahren hergestellt werden.

Die bei der Verfahrensvariante (c) weiter als Ausgangsstoffe zu verwendenden Sulfonsäurechloride sind durch die Formel (V) allgemein definiert. In Formel (V) hat $R^6$ vorzugsweise die gleiche Bedeutung, wie sie oben im Rahmen der Substituentendefinition der Formel (I) vorzugsweise angegeben ist.

Als Beispiele für die Ausgangsstoffe der Formel (V) können die Sulfonsäurechloride gelten, die oben als Beispiele für Verbindungen der Formel (III) genannt wurden; ferner
2-Chlor-, 3-Chlor-, 4-Chlor-, 2,5-Dichlor-, 2-Fluor-, 4-Fluor-, 2-Brom-, 4-Brom-, 2-Nitro-, 4-Nitro-, 2-Cyano-, 2-Methyl-, 4-Methyl-, 2-Chlormethyl-, 2-Trifluormethyl-, 2-Methoxy-, 2-Difluormethoxy-, 2-Trifluormethoxy-, 2-Methylthio-, 2-Methylthiomethyl-, 2-Methylsulfinylmethyl-, 2-Methylsulfonylmethyl-, 2-Dimethylaminosulfonyl-, 2-Diethylaminosulfonyl-, 2-Phenyl-, 2-Methoxycarbonyl-, 2-Ethoxycarbonyl-, 2-Propoxycarbonyl-, 2-Isopropoxycarbonyl-, 2-Butoxycarbonyl-, 2-Dimethylaminocarbonyl-, 2-Diethylaminocarbonyl-, 2-Phenoxy-, 2-Methyl-5-chlor-, 2-Chlor-5-trifluormethyl-, 2-Methylsulfonyl-, 2-Ethylsulfonyl-, 2-Chlor-4-trifluormethoxy-, 3-Chlor-4-trifluormethoxy-, 2-Trifluormethoxy-5-chlor-, 3,5-Dichlor-, 2-Difluormethylthio-und 2-Trifluormethylthiobenzolsulfonsäurechlorid, ferner Methan-, Chlormethan-, Trifluormethan-, Ethan-, 2-Chlorethan-, Ethen-, Propan-, Butan-, Perfluorbutan-und Perfluor-octan-sulfonsäurechlorid; Methyl-, Ethyl-, Propyl-, Isopropyl-, Cyclohexyl-, Dimethyl-und Diethyl-sulfamidsäurechlorid.

Die Sulfonsäurechloride der Formel (V) sind bekannt und/oder können nach an sich bekannten Verfahren hergestellt werden (vergl. Literatur und Synthesewege für die Verbindungen der Formel (III) - oben).

Die als Ausgangsstoffe für die Verfahrensvariante (d) zu verwendenden Sulfonylguanidin-Derivate sind durch die Formel (VI) allgemein definiert. In Formel (VI) haben $R^4$, $R^5$, $R^6$, X, Y und Z vorzugsweise die gleichen Bedeutungen, wie sie oben im Rahmen der Substituentendefinition der Formel (I) vorzugsweise angegeben sind.

Als Beispiele für die Verbindungen der Formel (VI) seien genannt:
N'-(4-Methyl-pyrimidin-2-yl)-,
N'-(4-Methoxy-6-methyl-pyrimidin-2-yl)-,
N'-(4-Ethoxy-6-methyl-pyrimidin-2-yl)-,
N'-(4-Chlor-6-methoxy-pyrimidin-2-yl)-,
N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)-und
N'-(4-Difluormethoxy-6-methyl-pyrimidin-2-yl)-
-N"-methoxy-,
-N"-ethoxy-,
-N"-isopropoxy-,
-N"-propoxy-,
-N"-butoxy-,
-N"-isobutoxy-,
-N"-sec.-butoxy-,
-N"-pentyloxy-,
-N"-octyloxy-,
-N"-allyloxy-,
-N"-(3-chlor-propoxy)-,
-N"-methoxycarbonylmethoxy-,
-N"-ethoxycarbonylmethoxy-,
-N"-(2-phenyl-ethoxy)-,
-N"-benzyloxy-,
-N"-benzolsulfonyl-,
-N"-(4-methyl-benzyloxy)-,
-N"-(4-fluor-benzyloxy)-,
-N"-(4-chlor-benzyloxy)-,
-N"-(4-nitro-benzyloxy)-,
-N"-(4-methoxycarbonyl-benzyloxy)-,
-N"-benzolsulfonyl-,
-N"-(2-chlor-benzolsulfonyl)-,
-N"-(3-chlor-benzolsulfonyl)-,
-N"-(4-chlor-benzolsulfonyl)-,
-N"-(2-fluor-benzolsulfonyl)-,
-N"-(4-fluor-benzolsulfonyl)-,
-N"-(2-brom-benzolsulfonyl)-,
-N"-(2-cyano-benzolsulfonyl)-,
-N"-(2-nitro-benzolsulfonyl)-,
-N"-(4-nitro-benzolsulfonyl)-,
-N"-(2-methyl-benzolsulfonyl)-,
-N"-(4-methyl-benzolsulfonyl)-,
-N"-(2-trifluormethyl-benzolsulfonyl)-,
-N"-(2-methoxy-benzolsulfonyl)-,
-N"-(2-methoxycarbonyl-benzolsulfonyl)-,

-N"-(2-chlor-3-thienylsulfonyl)-,
-N"-(3-chlor-2-thienylsulfonyl)-,
-N"-(2-methoxycarbonyl-3-thienylsulfonyl)-,
-N"-(2,5-dichlor-3-thienylsulfonyl)-,
-N"-(2-thienylsulfonyl)-,
-N"-(3-thienylsulfonyl)-,
-N"-(5-chlor-2-thienylsulfonyl)-,
-N"-(5-methoxycarbonyl-3-thienylsulfonyl)-und
-N"-(5-methoxycarbonyl-2-thienylsulfonyl)-guanidin;
ferner
N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'-(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-, N'-(4-Diethylamino-6-ethylthio-s-triazin-2-yl)-, N'-(2,6-Dimethyl-pyrimidin-4-yl)-und N'-(2,6-Dimethoxy-pyrimidin-4-yl)-N"-methoxy-, -N"-ethoxy-, -N"-propoxy-, -N"-isopropoxy-, -N"-butoxy-, -N"-isobutoxy-, -N"-sec.butoxy-, -N"-pentyloxy-, -N"-hexyloxy-, -N"-octyloxy-, -N"-allyloxy-, -N"-(3-chlor-propoxy)-, -N"-methoxycarbonylmethoxy-, -N"-ethoxycarbonylmethoxy-, -N"-(2-phenylethoxy)-; -N"-benzyloxy-, -N"-(4-methyl-benzyloxy)-, -N"-(4-fluor-benzyloxy)-, -N"-(4-chlor-benzyloxy)-, -N"-(4-nitro-benzyloxy)-und -N"-(4-methoxycarbonylbenzyloxy)-,
-N"-(2-chlor-3-thienylsulfonyl)-,
-N"-(3-chlor-2-thienylsulfonyl)-,
-N"-(2-methoxycarbonyl-3-thienylsulfonyl)-,
-N"-(2,5-dichlor-3-thienylsulfonyl)-,
-N"-(2-thienylsulfonyl)-,
-N"-(3-thienylsulfonyl)-,
-N"-(5-chlor-2-thienylsulfonyl)-,
-N"-(5-methoxycarbonyl-3-thienylsulfonyl)-und
-N"-(5-methoxycarbonyl-2-thienylsulfonyl)-guanidin.

Die Sulfonylguanidin-Derivate der Formel (VI) können analog Verfahren (a) durch Umsetzung von Guanidin-Derivaten der Formel (II) mit etwa äquimolaren Mengen von Sulfonsäurechloriden der Formel (V) hergestellt werden.

Die bei der Verfahrensvariante (d) weiter als Ausgangsstoffe zu verwendenden Sulfonsäurechloride sind durch die Formel (III) allgemein definiert. In Formel (III) haben $R^1$ und $R^2$ vorzugsweise die gleiche Bedeutungen, wie sie oben im Rahmen der Subsituentendefinition der Formel (I) vorzugsweise genannt sind.

Beispiele für die Sulfonsäurechloride der Formel (III) und Herstellungsverfahren hierfür wurden bereits weiter oben im Zusammenhang mit der Beschreibung der Ausgangsstoffe für Verfahren (a) und (c) angegeben.

Die bei der Verfahrensvariante (e) als Ausgangsstoffe zu verwendenden Sulfonylisothioharnstoff-Derivate sind durch die Formel (VII) allgemein definiert. In Formel (VII) haben $R^1$, $R^2$, $R^5$, X, Y und Z vorzugsweise die gleichen Bedeutungen, wie sie oben im Rahmen der Substituentendefinition der Formel -(I) als bevorzugt genannt sind und $R^{15}$ steht vorzugsweise für Methyl.

Als Beispiele für die Verbindungen der Formel (VII) seien genannt:
N'-(4-Chlor-6-methylthio-pyrimidin-2-yl)-,
N'-(4-Methyl-pyrimidin-2-yl)-,
N'-(4-Methoxy-6-methyl-pyrimidin-2-yl)-,
N'-(4-Ethoxy-6-methyl-pyrimidin-2-yl)-,
N'-(4-Chlor-6-methoxy-pyrimidin-2-yl)-,

N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)-und
N'-(4-Difluormethoxy-6-methyl-pyrimidin-2-yl)-,
-N"-(2-chlor-3-thienylsulfonyl)-,
-N"-(3-chlor-2-thienylsulfonyl)-,
-N"-(2-methoxycarbonyl-3-thienylsulfonyl)-,
-N"-(2,5-dichlor-3-thienylsulfonyl)-,
-N"-(2-thienylsulfonyl)-,
-N"-(3-thienylsulfonyl)-,
-N"-(5-chlor-2-thienylsulfonyl)-,
-N"-(5-methoxycarbonyl-3-thienylsulfonyl)-und
-N"-(5-methoxycarbonyl-2-thienylsulfonyl)-S-methylisothioharnstoff;
ferner
N'-(4,6-Dimethyl-s-triazin-2-yl)-, N'-(4-Methoxy-6-methyl-s-triazin-2-yl)-, N'-(4-Ethoxy-6-methyl-s-triazin-2-yl)-, N'-(4,6-Dimethoxy-s-triazin-2-yl)-, N'-(4,6-Diethoxy-s-triazin-2-yl)-, N'-(4-Ethoxy-6-methoxy-s-triazin-2-yl)-, N'-(4-Methyl-6-methylthio-s-triazin-2-yl)-, N'-(4-Ethylthio-6-methyl-s-triazin-2-yl)-, N'-(4-Methoxy-6-methylthio-s-triazin-2-yl)-, N'-(4-Ethoxy-6-methylthio-s-triazin-2-yl)-, N'-(4-Ethylthio-6-methoxy-s-triazin-2-yl)-, N'-(4-Ethoxy-6-ethylthio-s-triazin-2-yl)-, N'-(4,6-Bis-methylthio-s-triazin-2-yl)-, N'-(4,6-Bis-ethylthio-s-triazin-2-yl)-, N'-(4-Methyl-6-methylamino-s-triazin-2-yl)-, N'-(4-Ethylamino-6-methyl-s-triazin-2-yl)-, N'-(4-Dimethylamino-6-methyl-s-triazin-2-yl)-, N'-(4-Diethylamino-6-methyl-s-triazin-2-yl)-, N'-(4-Methoxy-6-methylamino-s-triazin-2-yl)-, N'-(4-Ethylamino-6-methoxy-s-triazin-2-yl)-, N'-(4-Dimethylamino-6-methoxy-s-triazin-2-yl)-, N'-(4-Diethylamino-6-methoxy-s-triazin-2-yl)-, N'-(4-Ethoxy-6-methylamino-s-triazin-2-yl)-, N'-(4-Ethoxy-6-ethylamino-s-triazin-2-yl)-, N'-(4-Dimethylamino-6-ethoxy-s-triazin-2-yl)-, N'-(4-Diethylamino-6-ethoxy-s-triazin-2-yl)-, N'-(4-Methylamino-6-methylthio-s-triazin-2-yl)-, N'-(4-Ethylamino-6-methylthio-s-triazin-2-yl)-, N'-(4-Dimethylamino-6-methylthio-s-triazin-2-yl)-, N'-(4-Diethylamino-6-methylthio-s-triazin-2-yl)-, N'-(4-Ethylthio-6-methylamino-s-triazin-2-yl)-, N'-(4-Dimethylamino-6-ethylthio-s-triazin-2-yl)-, N'-(4-Diethylamino-6-ethylthio-s-triazin-2-yl)-, N'-(2,6-Dimethyl-pyrimidin-4-yl)-und N'-(2,6-Dimethoxy-pyrimidin-4-yl)-
-N"-(2-chlor-3-thienylsulfonyl)-,
-N"-(3-chlor-2-thienylsulfonyl)-,
-N"-(2-methoxycarbonyl-3-thienylsulfonyl)-,
-N"-(2,5-dichlor-3-thienylsulfonyl)-,
-N"-(2-thienylsulfonyl)-,
-N"-(3-thienylsulfonyl)-,
-N"-(5-chlor-2-thienylsulfonyl)-,
-N"-(5-methoxycarbonyl-3-thienylsulfonyl)-und
-N"-(5-methoxycarbonyl-2-thienylsulfonyl)-S-methylisothioharnstoff.

Die Sulfonylisothioharnstoff-Derivate der Formel (VII) sind bekannt und/oder können nach an sich bekannten Verfahren hergestellt werden (vgl. EP-OS 5 986).

Man erhält die Verbindungen der Formel (VII), wenn man N-Sulfonyl-imino-dithiokohlensäureester der Formel (VIII)

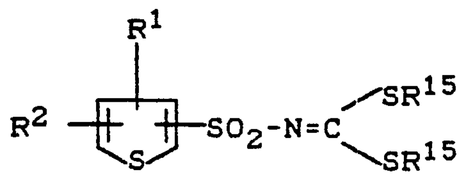

(VIII)

in welcher
$R^1$, $R^2$ und $R^{15}$ die oben angegebenen Bedeutungen haben,
mit Aminohetarenen der Formel (IX)

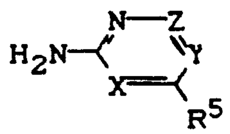

(IX)

in welcher

$R^5$, X, Y und Z die oben angegebenen Bedeutung haben,

gegebenenfalls in Gegenwart von Basen, wie z. B. Natriumhydrid oder Kalium-tert.-butylat, und gegebenenfalls in Gegenwart von Verdünnungsmitteln, wie z. B. Tetrahydrofuran, Dioxan oder Dimethylformamid, bei Temperaturen zwischen 0 °C und 100 °C umsetzt, nach Reaktionsende mit Wasser verdünnt, mit einer starken Säure, wie z. B. Salzsäure, ansäuert und die kristallin anfallenden Produkte der Formel (VII) durch Absaugen isoliert.

Die hierbei als Ausgangsstoffe benötigten N-Sulfonyliminodithiokohlensäureester sind durch die Formel (VIII) allgemein definiert. In Formel (VIII) haben $R^1$, $R^2$ und $R^{15}$ vorzugsweise die gleichen Bedeutungen, wie sie oben im Rahmen der Substituentendefinition für Formel (I) bzw. Formel (VII) vorzugsweise angegeben sind.

Als Beispiele für die Verbindungen der Formel (VIII) seien genannt:

N-(2-Chlor-3-thienylsulfonyl)-,
N-(3-Chlor-2-thienylsulfonyl)-,
N-(2-Methoxycarbonyl-3-thienylsulfonyl)-,
N-(2,5-Dichlor-3-thienylsulfonyl)-,
N-(2-Thienylsulfonyl)-,
N-(3-Thienylsulfonyl)-,
N-(5-Chlor-2-thienylsulfonyl)-,
N-(5-Methoxycarbonyl-3-thienylsulfonyl)-,
N-(5-Methoxycarbonyl-2-thienylsulfonyl)-und
N-(2-Ethoxycarbonyl-3-thienylsulfonyl)-S,S-dimethyl-iminodithiokohlensäureester.

Die N-Sulfonyl-imino-dithiokohlensäureester der Formel (VIII) sind bekannt und/oder können nach an sich bekannten Verfahren hergestellt werden (vergl. Chem. Ber. 99 (1966), 2855 und EP-OS 5 986).

Man erhält die Verbindungen der Formel (VIII), wenn man Sulfonsäureamide der Formel (X)

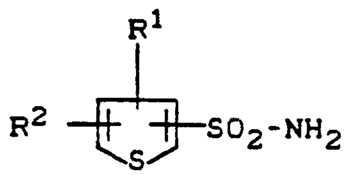

(X)

in welcher

$R^1$ und $R^2$ die oben angegebenen Bedeutungen haben,

mit Schwefelkohlenstoff in Gegenwart einer starken Base, wie z. B. Natriumhydroxid, und gegebenenfalls in Gegenwart von Verdünnungsmitteln, wie z. B. Wasser und Dimethylformamid, bei Temperaturen zwischen 0 °C und 100 °C umsetzt und anschließend (in situ) mit einem Alkylierungsmittel der Formel (XI)

$Q-R^{15}$ (XI)

in welcher

$R^{15}$ die oben angegebene Bedeutung hat und

Q für Chlor, Brom oder Iod steht,

bei Temperaturen zwischen 0 °C und 100 °C umsetzt.

Die nach Verdünnen mit Wasser kristallin anfallenden Produkte der Formel (VIII) können durch Absaugen isoliert werden.

Die als Ausgangsstoffe benötigten Sulfonsäureamide sind durch die Formel (X) allgemein definiert. In Formel (X) haben $R^1$ und $R^2$ vorzugsweise die gleichen Bedeutungen, wie sie oben im Rahmen der Substituentendefinition der Formel (I) als bevorzugt angegeben sind.

Als Beispiele für die Verbindungen der Formel (X) seien genannt:

2-Chlor-3-thienyl-, 3-Chlor-2-thienyl-, 2-Methoxycarbonyl-3-thienyl-, 2,5-Dichlor-3-thienyl-, 2-Thienyl-, 3-Thienyl-, 5-Chlor-2-thienyl-, 5-Methoxycarbonyl-3-thienyl-, 5-Methoxycarbonyl-2-thienyl-und 2-Ethoxycarbonyl-3-thienylsulfonamid.

Die Sulfonsäureamide der Formel (X) sind bekannt und/oder können nach an sich bekannten Verfahren hergestellt werden.

Man erhält diese Verbindungen auf an sich bekannte Weise durch Umsetzung von Sulfonsäurechloriden der Formel (III) -oben - mit Ammoniak, gegebenenfalls in Gegenwart von Verdünnungsmitteln, wie z. B. Diethylether, Tetrahydrofuran oder auch Wasser, bei Temperaturen zwischen 0 °C und 100 °C. Die hierbei kristallin anfallenden Produkte der Formel (X) können durch Absaugen isoliert werden.

Beispiele für geeignete Ausgangsstoffe der Formel (III) sind oben bei der Beschreibung der Ausgangsstoffe für Verfahren (a) aufgeführt.

Als Beispiele für die Ausgangsstoffe der Formel (XI) seien genannt:
Methylchlorid, Methylbromid, Methyliodid, Ethylchlorid, Ethylbromid und Ethyliodid sowie Benzylchlorid und Benzylbromid.

Die Verbindungen der Formel (XI) sind bekannt.

Die weiter als Ausgangsstoffe zu verwendenden Aminohetarene sind durch die Formel (IX) allgemein definiert. In Formel (IX) haben $R^5$, X, Y und Z vorzugsweise die gleichen Bedeutungen wie sie oben im Rahmen der Substituentendefinition der Formel (I) vorzugsweise angegeben sind.

Als Beispiele für die Verbindungen der Formel (IX) seien genannt:
4-Chlor-6-methoxy-, 4-Chlor-6-ethoxy-, 4-Chlor-6-dimethylamino-, 4-Methyl-6-methylthio-, 4-Dimethylamino-6-methyl-, 4-Difluormethoxy-6-methyl-, 4,6-Dimethoxy-, 4-Methoxy-6-methyl-, 4-Ethoxy-6-methyl-und 4-Methyl-2-amino-pyrimidin;
2,6-Dimethyl-und 2,6-Dimethoxy-4-amino-pyrimidin;
4,6-Dimethyl-, 4-Methoxy-6-methyl-, 4-Ethoxy-6-methyl-, 4,6-Dimethoxy-, 4,6-Diethoxy-, 4-Ethoxy-6-methoxy-, 4-Methyl-6-methylthio-, 4-Ethylthio-6-methyl-, 4-Methoxy-6-methylthio-, 4-Ethoxy-6-methylthio-, 4-Ethylthio-6-methoxy-, 4-Ethoxy-6-ethylthio-, 4,6-Bis-methylthio-, 4,6-Bis-ethylthio-, 4-Methyl-6-methylamino-, 4-Ethylamino-6-methyl-, 4-Dimethylamino-6-methyl-, 4-Diethylamino-6-methyl-, 4-Methoxy-6-methylamino-, 4-Ethylamino-6-methoxy-, 4-Dimethylamino-6-methoxy-, 4-Diethylamino-6-methoxy-, 4-Ethoxy-6-methylamino-, 4-Ethoxy-6-ethylamino-, 4-Dimethylamino-6-ethoxy-, 4-Diethylamino-6-ethoxy-, 4-Methylamino-6-methylthio-, 4-Ethylamino-6-methylthio-, 4-Dimethylamino-6-methylthio-, 4-Diethylamino-6-methylthio-, 4-Ethylthio-6-methylamino-, 4-Dimethyl-amino-6-ethylthio-und 4-Diethylamino-6-ethylthio-2-amino-s-triazin.

Die Aminohetarene der Formel (IX) sind bekannt und/oder können nach an sich bekannten Verfahren hergestellt werden.

Die bei der Verfahrensvariante (e) weiter als Ausgangsstoffe zu verwendenden Aminoverbindungen sind durch die Formel (IV) allgemein definiert. In Formel (IV) steht $R^3$ vorzugsweise für Wasserstoff oder Methyl und $R^4$ hat vorzugsweise die gleiche Bedeutung, wie sie oben im Rahmen der Substituentendefinition für Formel (I) vorzugsweise angegeben ist.

Beispiele für geeignete Ausgangsstoffe der Formel (IV) und Literaturangaben hierzu sind oben bei der Beschreibung der Ausgangsstoffe für Verfahren (b) aufgeführt.

Die bei der Verfahrensvariante (f) als Ausgangsstoffe zu verwendenden Verbindungen sind durch die Formel (I) -mit der Maßgabe, daß M für Wasserstoff steht -allgemein definiert. In Formel (I) -soweit sie die als Ausgangsstoffe für Verfahren (f) zu verwendenden Verbindungen betrifft -steht M für Wasserstoff und $R^1$, $R^2$, $R^3$, $R^4$, $R^5$, X, Y und Z haben vorzugsweise die gleichen Bedeutungen, wie sie oben im Rahmen der Substituentendefinition für Formel (I) vorzugsweise angegeben sind.

Die als Ausgangsstoffe für Verfahren (f) zu verwendenden Verbindungen der Formel (I) können nach den unter (a), (b), (c), (d) und (e) beschriebenen Verfahren hergestellt werden.

Als Beispiele für die bei Verfahren (f) zu verwendenden Metallhydroxide, -hydride, -alkanolate bzw. metallorganische Verbindungen seien genannt:
Lithium-, Natrium-, Kalium-, Magnesium-und Calcium-hydroxid, Lithium-, Natrium-und Calcium-hydrid, Natrium-methanolat und -ethanolat, Kalium-methanolat, -ethanolat und Kalium-tert.-butanolat sowie Butyllithium und Isopropylmagnesiumchlorid.

Die bei der Verfahrensvariante (g) als Ausgangsstoffe zu verwendenden Verbindungen sind durch die Formel (I) -mit der Maßgabe, daß M für Wasserstoff steht -allgemein definiert. In Formel (I) soweit sie die als Ausgangsstoffe für Verfahren (g) zu verwendenden Verbindungen betrifft -steht M für Wasserstoff und $R^1$, $R^2$, $R^3$, $R^4$, $R^5$, X, Y und Z haben vorzugsweise die gleichen Bedeutungen, wie sie oben im Rahmen der Substituentendefinition für Formel (I) vorzugsweise angegeben sind.

Die als Ausgangsstoffe für Verfahren (g) zu verwendenden Verbindungen der Formel (I) können nach den unter (a), (b), (c), (d) und (e) beschriebenen Verfahren hergestellt werden. Bei Verfahren (g) werden starke Säuren als Ausgangsstoffe eingesetzt. Vorzugsweise sind dies Halogenwasserstoffsäuren wie Hydrogenchlorid, Hydrogenbromid oder Hydrogeniodid, weiter Schwefelsäure oder gegebenenfalls durch Fluor

oder Chlor substituierte Alkansulfonsäuren mit bis zu 4 Kohlenstoffatomen wie z. B. Methansulfonsäure, Ethansultonsäure, Chlormethansulfonsäure, 2-Chlorethansulfonsäure und Trifluormethansulfonsäure, Trifluoressigsäure, ferner Benzolsulfonsäure, p-Toluolsulfonsäure, Naphthalin-1-sulfonsäure, Naphthalin-2-sulfonsäure, Naphthalin-1,4-, -1,5-, -1,6-, -2,6-und -2,7-disulfonsäure.

Das erfindungsgemäße Verfahren (a) zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Be tracht. Hierzu gehören gegebenenfalls halogensubstituierte Kohlenwasserstoffe wie z. B. Methylenchlorid, Chloroform, 1,2-Dichlorethan, Toluol, Xylol und Chlorbenzol, Nitrile wie z. B. Acetonitril und Propionitril, Ether wie z. B. 1,2-Dimethoxyethan, Tetrahydrofuran und Dioxan sowie Dimethylformamid, Dimethylacetamid, Dimethylsulfoxid, Sulfolan, Pyridin und 2-Methyl-5-ethyl-pyridin.

Als Säureakzeptoren können bei Verfahren (a) praktisch alle üblicherweise verwendeten Säurebindemittel eingesetzt werden. Hierzu gehören insbesondere Alkalimetall-und Erdalkalimetallhydroxide, Alkalimetall-und Erdalkalimetallhydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicyclononen (DBN), Diazabicycloundecen - (DBU), Pyridin, 2-Methyl-5-ethyl-pyridin und 4-Dimethylamino-pyridin.

Die Reaktionstemperaturen können bei Verfahren (a) innerhalb eines größeren Bereiches variiert werden. Im allgemeinen arbeitet man zwischen -80 °C und +100 °C, vorzugsweise zwischen -30 °C und +50 °C. Das erfindungsgemäße Verfahren (a) wird im allgemeinen bei Normaldruck durchgeführt.

Zur Durchführung von Verfahren (a) setzt man je Mol Guanidin-Derivat der Formel (II) im allgemeinen zwischen 1 und 5 Mol, vorzugsweise zwischen 2 und 3 Mol Sulfonsäurechlorid der Formel (III) ein.

Die Reaktionskomponenten werden gewöhnlich bei Raumtemperatur oder unter Außenkühlung zusammengegeben und das Reaktionsgemisch wird bis zum Reaktionsende gerührt.

Die Aufarbeitung und Isolierung der neuen Verbindungen erfolgt nach üblichen Methoden: Gegebenenfalls nach Abdestillieren flüchtiger Komponenten wird mit Wasser und einem mit Wasser nicht mischbaren Lösungsmittel, wie z. B. Methylenchlorid, Chloroform oder Toluol, geschüttelt, die organische Phase mit Wasser gewaschen, getrocknet, filtriert und eingeengt. Die im Rückstand verbleibenden Produkte der Formel (I) werden durch Digerieren mit organischen Lösungsmitteln, wie z. B. Diethylether, Essigester, Ethanol oder Isopropanol zur Kristallisation gebracht und gegebenenfalls durch Umkristallisieren gereinigt.

Das erfindungsgemäße Verfahren (b) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n-und i-Propanol, Ether, wie Tetrahydrofuran, Dioxan und 1,2-Dimethoxyethan, Ester, wie Essigsäuremethylester und -ethylester, Nitrile, wie z. B. Acetonitril oder Propionitril, sowie Dimethylformamid und Wasser.

Als Säureakzeptoren können bei Verfahren (b) Säurebindemittel eingesetzt werden, welche in ihren nucleophilen Eigenschaften nicht nennenswert mit den Aminoverbindungen der Formel (IV) konkurrieren. Als solche seien Alkalimetall-und Erdalkalimetallcarbonate, wie z. B. Kaliumcarbonat und Calciumcarbonat, tertiäre Amine, wie z. B. Triethylamin, N,N-Dimethylanilin und N,N-Dimethylbenzylamin, sowie Stickstoffheterocyclen, wie z. B. Pyridin, Diazabicyclooctan (DABCO) und Diazabicycloundecen (DBU) genannt.

Die Reaktionstemperatur kann bei Verfahren (b) innerhalb eines größeren Bereiches variiert werden. Im allgemeinen arbeitet man zwischen 0 °C und 150 °C, vorzugsweise zwischen 10 °C und 100 °C. Verfahren (b) wird im allgemeinen bei Normaldruck durchgeführt.

Zur Durchführung des erfindungsgemäßen Verfahrens (b) setzt man je Mol Verbindung der Formel (ID) im allgemeinen zwischen 1 und 10 Mol, vorzugsweise zwischen 2 und 5 Mol Aminoverbindung der Formel - (IV) oder deren Hydrochlorid ein.

Im allgemeinen wird die Verbindung der Formel (ID) mit dem Verdünnungsmittel bei 20 °C oder unter leichtem Kühlen versetzt und die Aminoverbindung der Formel (IV) bzw. das Hydrochlorid hiervon und gegebenenfalls ein geeigneter Säureakzeptor dazugegeben. Das Reaktionsgemisch wird dann im allgemeinen bei Raumtemperatur oder erhöhter Temperatur bis zum Reaktionsende gerührt.

Die Aufarbeitung kann nach üblichen Methoden duchgeführt werden. Soweit die Produkte der Formel - (I) aus dem Reaktionsgemisch kristallin anfallen, können sie durch Absaugen isoliert werden. Anderenfalls wird -gegebenenfalls nach Einengen -mit Wasser verdünnt und mit einem mit Wasser praktisch nicht mischbaren Lösungsmittel, wie z. B. Methylenchlorid, extrahiert. Durch Waschen der Extraktionslösung mit Wasser, Trocknen, Filtrieren, Einengen des Filtrates und Umkristallisieren des Rückstandes können die Produkte der Formel (I) in reiner Form erhalten werden.

Das erfindungsgemäße Verfahren (c) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (c) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (c) innerhalb eines größeren Bereichs variiert wrden. Im allgemeinen arbeitet man zwischen -80 °C und +100 °C, vorzugsweise zwischen -30 °C und +50 °C. Das Verfahren (c) wird im allgemeinen bei Normaldruck durchgeführt.

Zur Durchführung von Verfahren (c) setzt man je Mol Thienylsulfonylguanidin-Derivat der Formel (IE) im allgemeinen zwischen 1 und 2 Mol, vorzugsweise zwischen 1,1 und 1,5 Mol Sulfonsäurechlorid der Formel -(V) ein.

Die Durchführung und Aufarbeitung kann bei Verfahren (c) analog zu Verfahren (a) erfolgen.

Das erfindungsgemäße Verfahren (d) wird vorzugsweise unter Verwendung von Verdünnungsmitteln durchgeführt. Als solche kommen praktisch alle inerten organischen, vorzugsweise jedoch aprotisch polare Solventien in Betracht, wie sie oben im Zusammenhang mit der Beschreibung des erfindungsgemäßen Verfahrens (a) genannt wurden.

Als Säureakzeptoren können bei Verfahren (d) praktisch alle üblicherweise verwendeten Säurebindemittel eingesetzt werden. Hierzu gehören insbesondere die oben im Zusammenhang mit der Beschreibung des erfindungsgemäßen Verfahrens (a) genannten Säurebindemittel.

Die Reaktionstemperaturen können bei Verfahren (d) innerhalb eines größeren Bereichs variiert werden. Im allgemeinen arbeitet man zwischen -80 °C und +100 °C, vorzugsweise zwischen -30 °C und +50 °C. Das Verfahren (d) wird im allgemeinen bei Normaldruck durchgeführt.

Zur Durchführung von Verfahren (d) setzt man je Mol Sulfonylguanidin-Derivat der Formel (VI) im allgemeinen zwischen 1 und 2 Mol, vorzugsweise zwischen 1,1 und 1,5 Mol Sulfonsäurechlorid der Formel -(III) ein.

Die Durchführung und Aufarbeitung kann bei Verfahren (d) analog zu Verfahren (a) erfolgen.

Das erfindungsgemäße Verfahren (e) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören insbesondere Tetrahydrofuran, Dioxan, 1,2-Dimethoxyethan, Acetonitril, Aceton, Methylethylketon, Methylisobutylketon, Dimethylformamid, Dimethylacetamid und Dimethylsulfoxid.

Die Reaktionstemperatur kann bei Verfahren (e) innerhalb eines größeren Bereichs variiert werden. Im allgemeinen arbeitet man zwischen 0 °C und 150 °C, vorzugsweise zwischen 10 °C und 100 °C. Das Verfahren (e) wird im allgemeinen bei Normaldruck oder geringfügig vermindertem Druck durchgeführt.

Zur Durchführung des erfindungsgemäßen Verfahrens (e) setzt man je Mol Sulfonylisothioharnstoff-Derivat der Formel (VII) im allgemeinen zwischen 1 und 10 Mol, vorzugsweise zwischen 1 und 5 Mol Aminoverbindung der Formel (IV) ein.

Im allgemeinen werden die Sulfonylisothioharnstoff-Derivate der Formel (VII) und das Verdünnungsmittel vorgelegt und die Aminoverbindung der Formel (IV) wird zudosiert. Das Reaktionsgemisch wird dann bis zum Reaktionsende gerührt. Die Produkte der Formel (I) fallen gewöhnlich nach Abkühlen und gegebenenfalls nach Ansäuern, z. B. mit Salzsäure, kristallin an und können durch Absaugen isoliert werden.

Das erfindungsgemäße Verfahren (f) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie z. B. Ethanol, n-und iso-Propanol, Ether, wie z. B. Tetrahydrofuran, Dioxan und 1,2-Dimethoxyethan, Ester, wie z. B. Esigsäureethylester und -methylester sowie Nitrile, wie z. B. Acetonitril.

Die Reaktionstemperatur kann bei Verfahren (f) innerhalb eines größeren Bereiches variiert werden. Im allgemeinen arbeitet man zwischen -20 °C und +50 °C, vorzugsweise zwischen 0 °C und 30 °C. Verfahren (f) wird im allgemeinen bei Normaldruck durchgeführt.

Zur Durchführung des erfindungsgemäßen Verfahrens (f) setzt man je Mol Verbindung der Formel (I) im allgemeinen zwischen 0,9 und 1,2 Mol, vorzugsweise zwischen 0,95 und 1,1 Mol Metallverbindung ein.

Im allgemeinen werden die Verbindungen der Formel (I) und das Verdünnungsmittel vorgelegt und -gegebenenfalls unter leichter Außenkühlung -die Metallverbindung -gegebenenfalls im Verdünnungsmittel gelöst -zudosiert. Das Reaktionsgemisch wird bis zum Reaktionsende gerührt. Die salzartigen Produkte der Formel (I) fallen im allgemeinen kristallin an und können durch Absaugen isoliert werden.

Das erfindungsgemäße Verfahren (g) wird vorzugsweise unter Verwendung von Verdünnungsmitteln durchgeführt. Als solche kommen praktisch alle inerten organischen Lösungsmittel in Betracht. Hierzu gehören insbesondere Alkohole, wie Methanol, Ethanol, n-und iso-Propanol, Ether, wie Tetrahydrofuran, Dioxan und 1,2-Dimethoxyethan, Ester, wie Essigsäuremethylester und -ethylester sowie Ketone, wie Aceton, Methylethylketon und Methylisobutylketon.

Soweit die als Ausgangsstoffe verwendeten Säuren in wässriger Lösung eingesetzt werden, kann vorteilhaft auch Essigsäureanhydrid als Verdünnungsmittel verwendet werden.

Die Reaktionstemperatur kann bei Verfahren (g) innerhalb eines größeren Bereiches variiert werden. Im allgemeinen arbeitet man zwischen -20 °C und +50 °C, vorzugsweise zwischen 0 °C und 30 °C. Verfahren (g) wird im allgemeinen bei Normaldruck durchgeführt.

Zur Durchführung des erfindungsgemäßen Verfahrens (g) setzt man je Mol Verbindung der Formel (I) im allgemeinen zwischen 1 und 10 Mol, vorzugsweise zwischen 1 und 5 Mol einer starken Säure ein.

Im allgemeinen werden die Verbindungen der Formel (I) und das Verdünnungsmittel vorgelegt und -gegebenenfalls unter leichter Außenkühlung -die starke Säure zudosiert. Das Reaktionsgemisch wird bis zum Reaktionsende gerührt. Die 1:1-Addukte fallen im allgemeinen kristallin an und können durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapsis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe können zur selektiven Bekämpfung mono-und dikotyler Unkräuter in Sojabohnenkulturen, insbesondere im Vorauflaufverfahren eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder -schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Monmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln: als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethylharnstoff, 3-(3-Chlor-4-methylphenyl)-1,1-dimethylharnstoff, 3-(4-Isopropyl-phenyl)-1,1-dimethylharnstoff, 4-Amino-6-(1,1-dimethyl-ethyl)-3-methylthio-1,2,4-triazin-5-(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5-(4H)-on, 2-Chlor-4-ethylamino-6-isopropylamino-1,3,5-triazin, das R-Enantiomere des 2-[4-(3,5-Dichlorpyridyl-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxyessigsäure, 2-(2-Methyl-4-chlorphenoxy)-propionsäure, 3,5-Diiod-4-hydroxy-benzonitril, 3,5-Dibrom-4-hydroxybenzonitril sowie Diphenylether und Phenylpyridazine, wie z.B. Pyridate. Einige Mischungen zeigen überraschenderweise auch synergistische Wirkung. Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

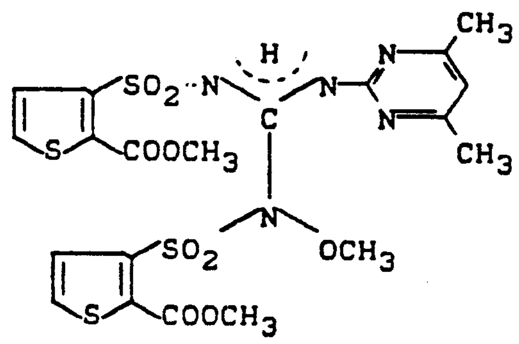

(Verfahren (a))

Eine Lösung von 50,4 g (0,45 Mol) Diazabicyclooctan in 100 ml Methylenchlorid wird zu einer auf -10 °C gekühlten Mischung aus 19,5 g (0,1 Mol) N'-(4,6-Dimethyl-pyrimidin-2-yl)-N''-methoxy-guanidin, 96,2 g - (0,4 Mol) 2-Methoxycarbonyl-3-thienylsulfonsäurechlorid und 300 ml Methylenchlorid unter Rühren getropft. Das Reaktionsgemisch wird noch 2 Stunden bei -10 °C bis 0 °C und anschließend 15 Stunden bei 20 °C gerührt. Das Reaktionsgemisch wird mit 50 ml 1 N Salzsäure und mit Wasser gewaschen, über Natriumsulfat getrocknet und eingeengt. Der Rückstand wird aus Essigsäureethylester umgelöst und getrocknet.

Man erhält 37,5 g (62 % der Theorie) N'-(4,6-Dimethyl-pyrimidin-2-yl)-N''-methoxy-N'',N'''-bis-(2-methoxycarbonyl-3-thienylsulfonyl)-guanidin vom Schmelzpunkt 120 -122 °C.

Beispiel 2

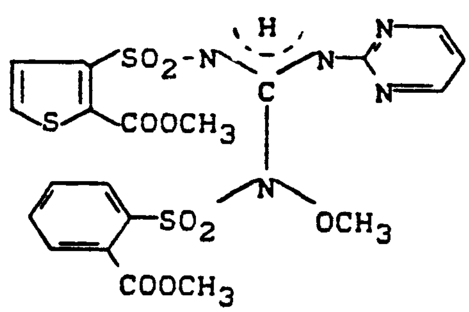

(Verfahren (d))

6,0 g (0,0164 Mol) N'-(Pyrimidin-2-yl)-N''-methoxy-N''-(2-methoxycarbonylbenzolsulfonyl)-guanidin werden in 100 ml Methylenchlorid gelöst, 7,9 g (0,033 Mol) 2-Methoxycarbonyl-3-thienylsulfonsäurechlorid werden zugegeben und die Mischung wird auf -20 °C abgekühlt. Zu dieser Mischung wird bei -20 °C eine Lösung von 3,7 g (0,033 Mol) Diazabicyclooctan in 20 ml Methylenchlorid getropft und anschließend 5 Stunden bei -20 °C und weitere 15 Stunden bei 20 -25 °C gerührt. Man wäscht das Reaktionsgemisch mit 50 ml 1N-Salzsäure und danach mit Wasser. Die Methylenchloridphase wird über Natriumsulfat getrocknet, filtriert und eingeengt. Der verbleibende Rückstand wird durch Chromatographie über eine Kieselgelsäule mit Essigsäureethylester als Laufmittel gereinigt.

Man erhält 0,75 g (8 % der Theorie) N'-(Pyrimidin-2-yl)-N''-methoxy-N''-(2-methoxycarbonylbenzolsulfonyl)-N'''-(2-methoxycarbonyl-3-thienylsulfonyl)-guanidin in Form hellgelber Kristalle vom Schmelzpunkt 67 °C (Zers.).

Analog können die in der nachstehenden Tabelle 1 aufgeführten Verbindungen der Formel (I) hergestellt werden:

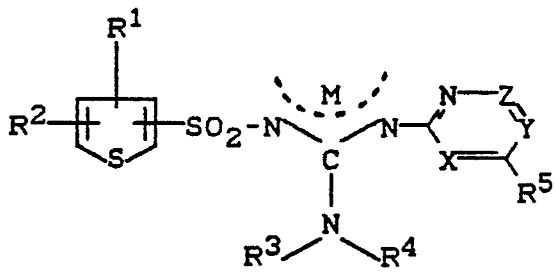 (I)

M = Wasserstoff

Tabelle 1

| Beisp.-Nr. | 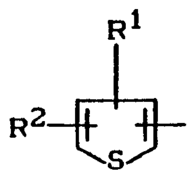 | $R^3$ | $R^4$ | 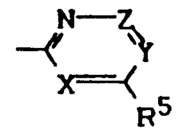 | Schmelzpunkt [°C] |
| --- | --- | --- | --- | --- | --- |
| 3 | 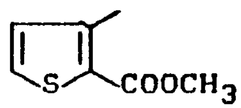 | 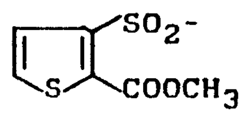 | $-OCH_3$ | 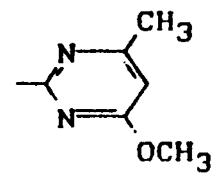 | 162 |
| 4 | 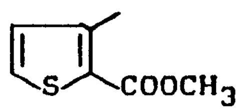 | 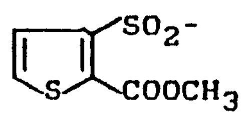 | $-OCH_3$ | 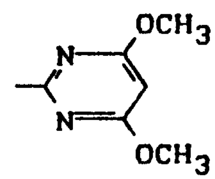 | |
| 5 | 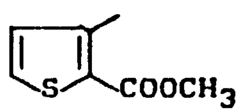 | 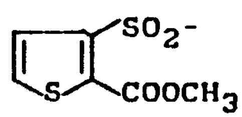 | $-OCH_3$ | 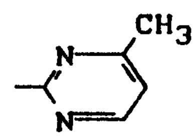 | |
| 6 | 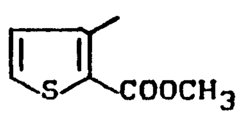 | 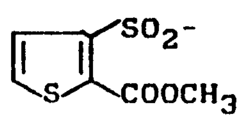 | $-OCH_3$ | 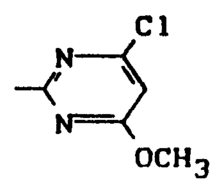 | |

Tabelle 1 - Fortsetzung

| Beisp.- Nr. | 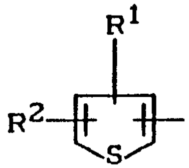 | $R^3$ | $R^4$ | 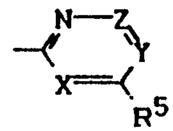 | Schmelzpunkt [°C] |
|---|---|---|---|---|---|
| 7 | 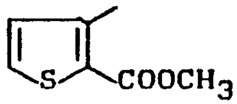 | 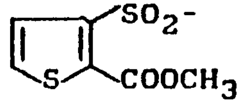 | $-OCH_3$ | 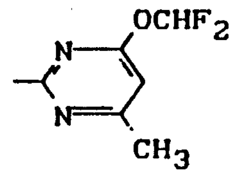 | |
| 8 | 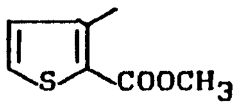 | 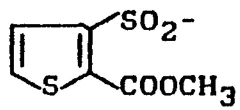 | $-OCH_3$ | 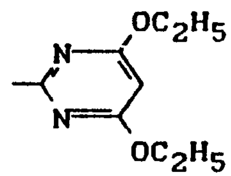 | |
| 9 | 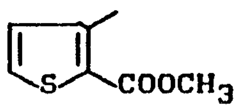 | 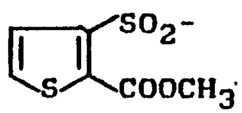 | $-OCH_3$ | 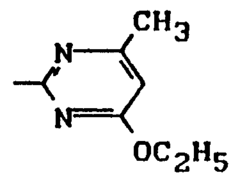 | |
| 10 | 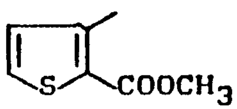 | 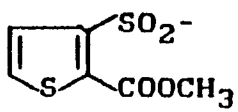 | $-OCH_3$ | 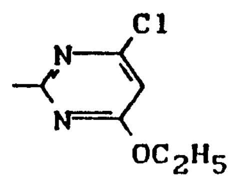 | |

Tabelle 1 - Fortsetzung

| Beisp.-Nr. | 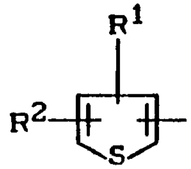 | $R^3$ | $R^4$ | 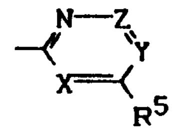 | Schmelzpunkt [°C] |
|---|---|---|---|---|---|
| 11 | 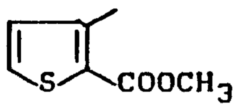 | 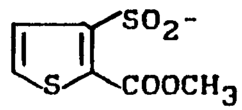 | $-OCH_3$ | 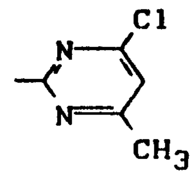 | |
| 12 | 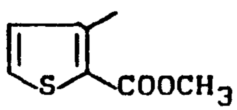 | H | $-OCH_3$ | 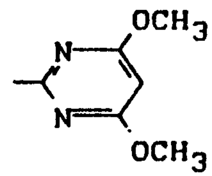 | |
| 13 | 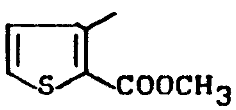 | H | $-N(CH_3)_2$ | 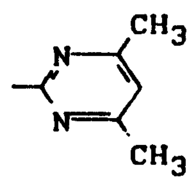 | |
| 14 | 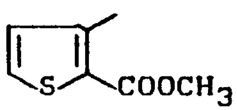 | H | 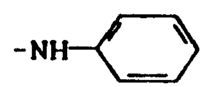 | 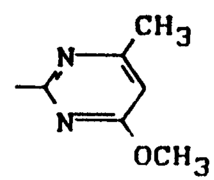 | |

<u>Tabelle 1</u> - Fortsetzung

| Beisp.-Nr. | 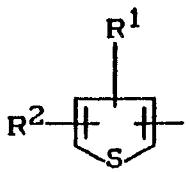 | $R^3$ | $R^4$ | 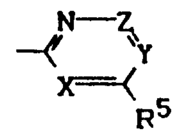 | Schmelzpunkt [°C] |
|---|---|---|---|---|---|
| 15 | 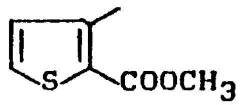 | 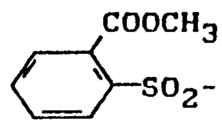 | $-OCH_3$ | 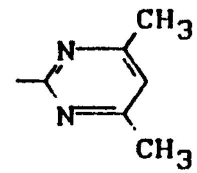 | |
| 16 | 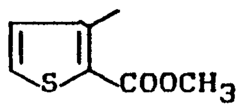 | 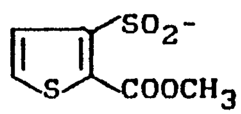 | $-OCH_2CH(CH_3)_2$ | 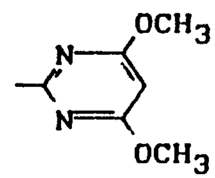 | |
| 17 | 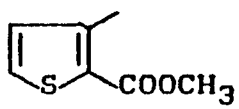 | 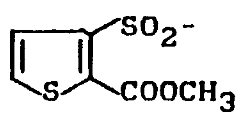 | $-OCH_2CH{=}CH_2$ | 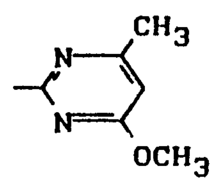 | |
| 18 | 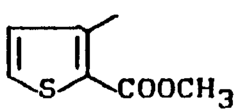 | $CH_3SO_2-$ | $-OC_2H_5$ | 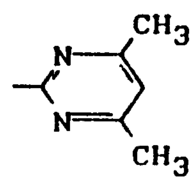 | |

Tabelle 1 - Fortsetzung

| Beisp.-Nr. | 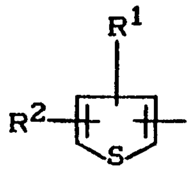 | $R^3$ | $R^4$ | 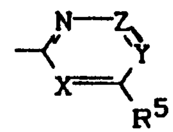 | Schmelzpunkt [°C] |
|---|---|---|---|---|---|
| 19 | 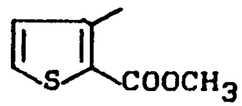 | 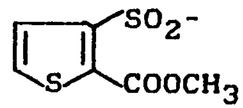 | $-OCH_3$ | 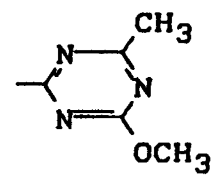 | 133 |
| 20 | 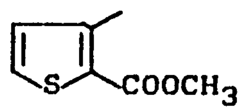 | 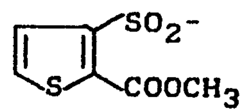 | $-OCH_3$ | 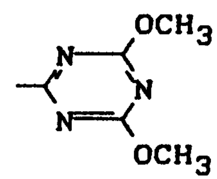 | 102 |
| 21 | 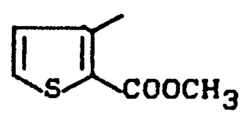 | 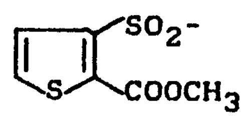 | $-OCH_3$ | 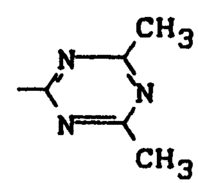 | |
| 22 | 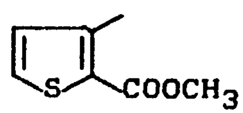 | 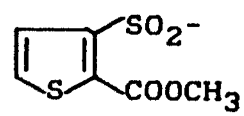 | $-OCH_3$ | 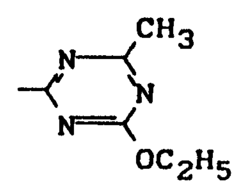 | |

<u>Tabelle 1</u> - Fortsetzung

| Beisp.-Nr. | 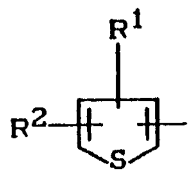 | $R^3$ | $R^4$ | 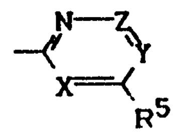 | Schmelzpunkt [°C] |
|---|---|---|---|---|---|
| 23 | 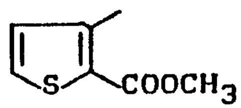 | H | $-OCH_3$ | 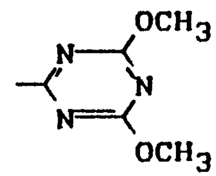 | |
| 24 | 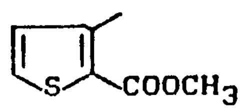 | H | $-N(CH_3)_2$ | 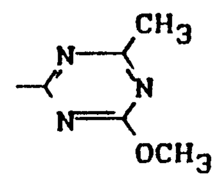 | |
| 25 | 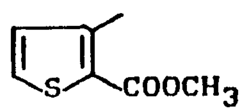 | 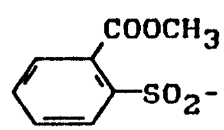 | $-OCH_3$ | 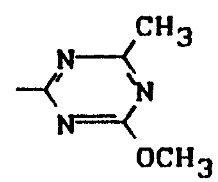 | |
| 26 | 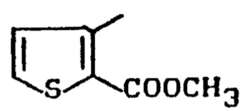 | H | $-OCH_2-$ 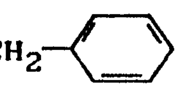 | 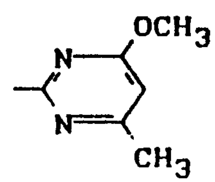 | |

Tabelle 1 - Fortsetzung

| Beisp.-Nr. | 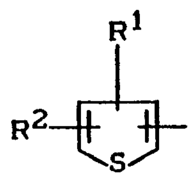 | $R^3$ | $R^4$ | 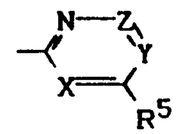 | Schmelzpunkt [°C] |
|---|---|---|---|---|---|
| 27 | 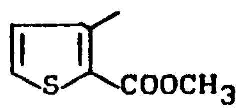 | H | $-NH_2$ | 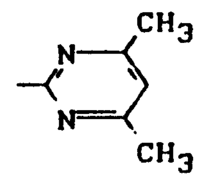 | |
| 28 | 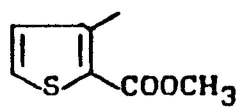 | $-CH_3$ | $-OCH_3$ | 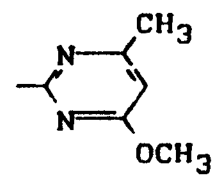 | |
| 29 | 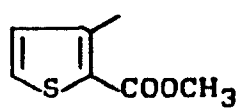 | H | $-NHCOCH_3$ | 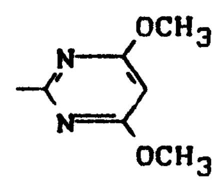 | |
| 30 | 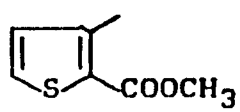 | 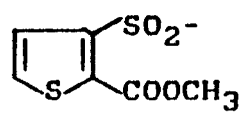 | $-OCH(CH_3)_2$ | 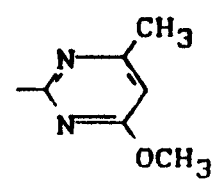 | |

Tabelle 1 - Fortsetzung

| Beisp.-Nr. | 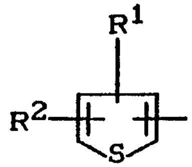 | $R^3$ | $R^4$ | 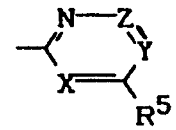 | Schmelzpunkt [°C] |
|---|---|---|---|---|---|
| 31 | 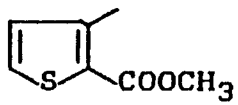 | 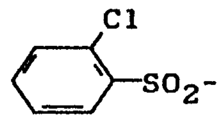 | $-OCH_3$ | 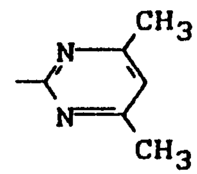 | |
| 32 | 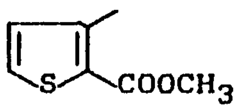 | 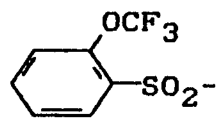 | $-OCH_3$ | 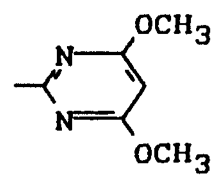 | |
| 33 | 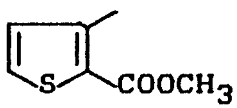 | H | $-OCH_2COOC_2H_5$ | 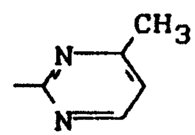 | |
| 34 | 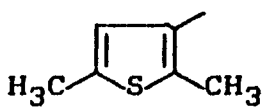 | 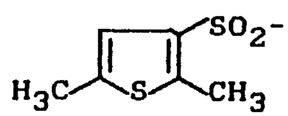 | $-OCH_3$ | 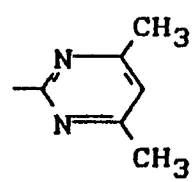 | 175 |
| 35 | 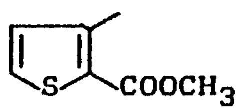 | H | $-OCH_3$ | 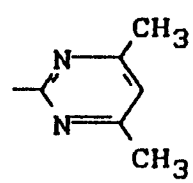 | 165 |

Herstellung von Ausgangsstoffen der Formel (II)

Beispiel (II-1)

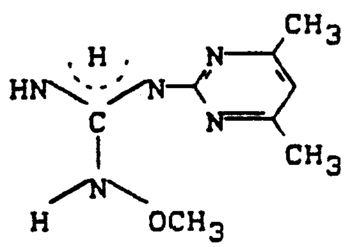

Eine Mischung aus 109 g (0,67 Mol) 0-Methylhydroxylamin-Hydrochlorid, 99 g (0,67 Mol) 2-Cyanamino-4,6-dimethylpyrimidin und 600 ml Ethanol wird 7 Stunden unter Rückfluß zum Sieden erhitzt. Anschließend wird der Alkohol im Wasserstrahlvakuum abdestilliert, der Rückstand in heißem Wasser gelöst und diese Lösung zu 100 ml konzentriertem Ammoniak gegeben. Das auskristallisierte Produkt wird abgesaugt und aus Ethanol umkristallisiert.

Man erhält 71,8 g (55 % der Theorie) N'-(4,6-Dimethylpyrimidin-2-yl)-N''-methoxy-guanidin vom Schmelzpunkt 134 °C bis 136 °C.

Analog können die in der nachstehenden Tabelle 2 aufgeführten Verbindungen der Formel (II) hergestellt werden:

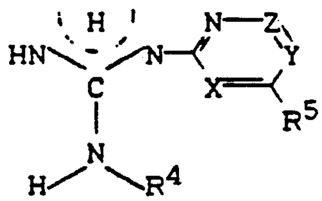

(II)

Tabelle 2

| Beisp.-Nr. | $R^4$ | 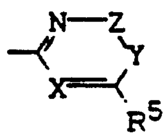 | Schmelzpunkt [°C] bzw. Brechungsindex |
|---|---|---|---|
| (II-2) | $-OCH_2CH(CH_3)_2$ | 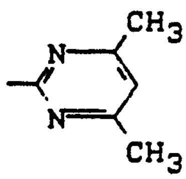 | 52 |
| (II-3) | $-OCH_2CH=CH_2$ | 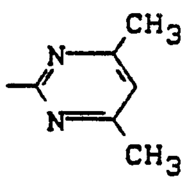 | 103 |
| (II-4) | $-OCH(CH_3)_2$ | 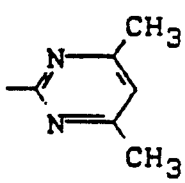 | 98 |
| (II-5) | $-OCH_2CH_2-$ 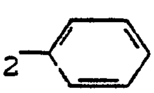 | 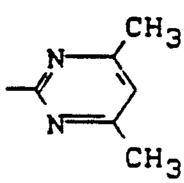 | $n_D^{24}$ = 1.5776 |

Tabelle 2 - Fortsetzung

| Beisp.-Nr. | $R^4$ | 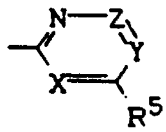 | Schmelzpunkt [°C] bzw. Brechungsindex |
|---|---|---|---|
| (II-6) | $-OCH-CH_2CH_3$<br>\|<br>$CH_3$ | 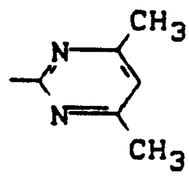 | 78 |
| (II-7) | $-OC_8H_{17}$(-n) | 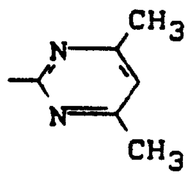 | 58 |
| (II-8) | $-OCH_2$ 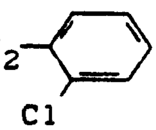 | 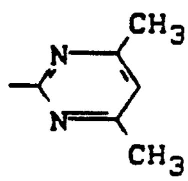 | 102 - 103 |
| (II-9) | $-OCH_2CH_2CH_2Cl$ | 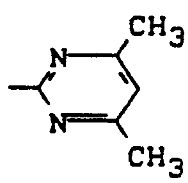 | 137 |
| (II-10) | 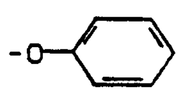 | 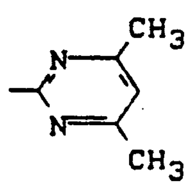 | 193 (Zers.) |
| (II-11) | $-OCH_2-COOCH_3$ | 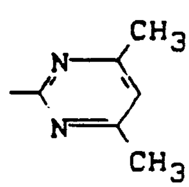 | 148 - 149 |

Tabelle 2 - Fortsetzung

| Beisp.-Nr. | $R^4$ | 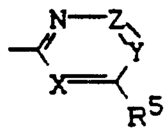 | Schmelzpunkt [°C] bzw. Brechungs-index |
|---|---|---|---|
| (II-12) | $-OCH_2-COOC_2H_5$ | 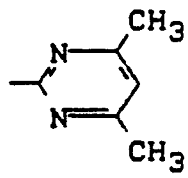 | 98 - 99 |
| (II-13) | 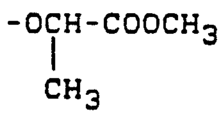 | 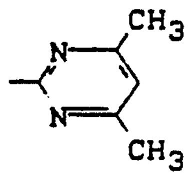 | 147 - 148 |
| (II-14) | 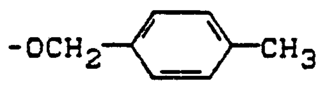 | 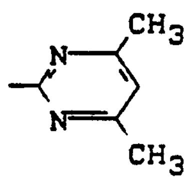 | 85 - 86 |
| (II-15) | 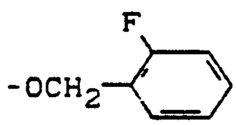 | 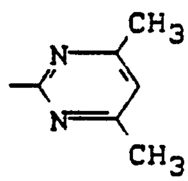 | 114 - 116 |
| (II-16) | 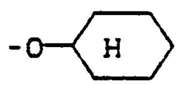 | 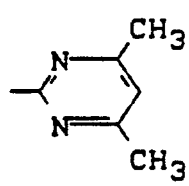 | |
| (II-17) | 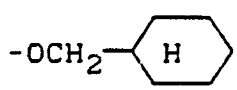 | 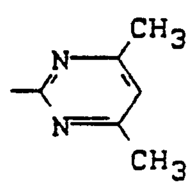 | 103 |

<u>Tabelle 2</u> - Fortsetzung

| Beisp.-Nr. | $R^4$ | 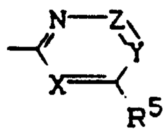 | Schmelzpunkt [°C] bzw. Brechungsindex |
|---|---|---|---|
| (II-18) | $-OCH_2CON(CH_3)_2$ | 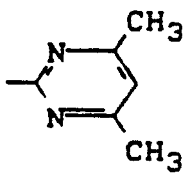 | |
| (II-19) | $-OCH_2OCH_3$ | 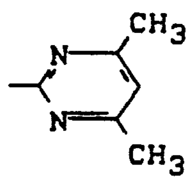 | |
| (II-20) | $-OCH_2SCH_3$ | 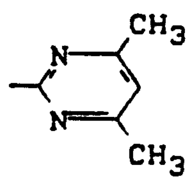 | |
| (II-21) | $-OCH_2-$ 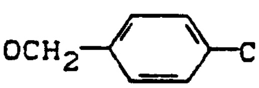 $-COOC_2H_5$ | 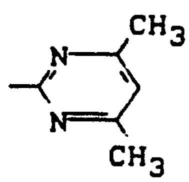 | 138 |
| (II-22) | $-OCH_2CF_3$ | 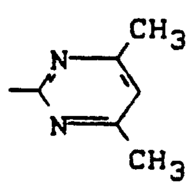 | |
| (II-23) | $-OCH_2-$ 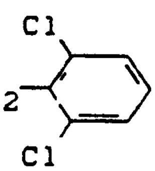 | 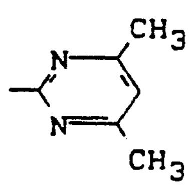 | 152 |

Tabelle 2 - Fortsetzung

| Beisp.-Nr. | $R^4$ | 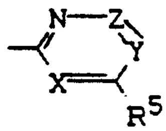 | Schmelzpunkt [°C] bzw. Brechungsindex |
|---|---|---|---|
| (II-24) | 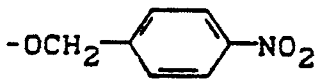 | 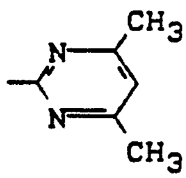 | 170 - 172 |
| (II-25) | $-OC_4H_9(-n)$ | 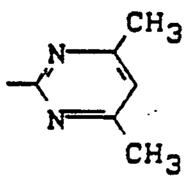 | $n_D^{20}$ = 1.5513 |
| (II-26) | $-OC_3H_7(-n)$ | 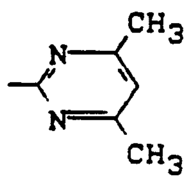 | 53 - 54 |
| (II-27) | $-OCH_2-COOC_3H_7(-i)$ | 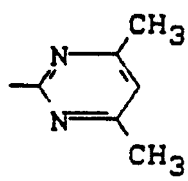 | 112 |
| (II-28) | 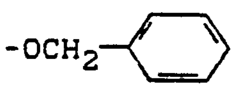 | 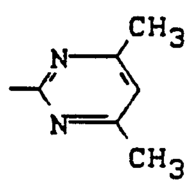 | 102 |
| (II-29) | $-OC_2H_5$ | 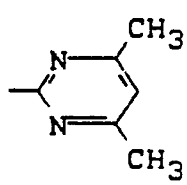 | 88 |

Tabelle 2 - Fortsetzung

| Beisp.-Nr. | $R^4$ | 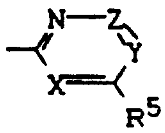 | Schmelzpunkt [°C] bzw. Brechungsindex |
|---|---|---|---|
| (II-30) | $-OCH_3$ | 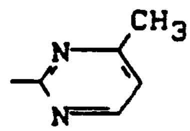 | 152 |
| (II-31) | $-OCH_3$ | 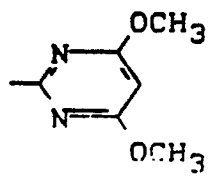 | 122 |
| (II-32) | $-OCH_3$ | 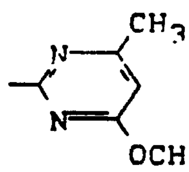 | 126 |
| (II-33) | $-OCH_3$ | 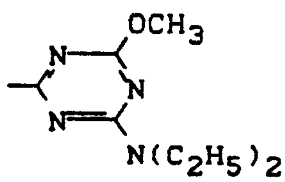 | 112 |
| (II-34) | $-OCH_3$ | 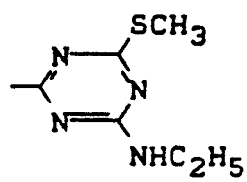 | 117 |
| (II-35) | $-OCH_2-CH(CH_3)_2$ | 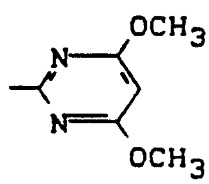 | 76 |

<u>Tabelle 2</u> - Fortsetzung

| Beisp.-Nr. | $R^4$ | 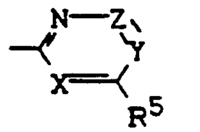 | Schmelzpunkt [°C] bzw. Brechungsindex |
|---|---|---|---|
| (II-36) | $-OCH-CH_2CH_3$<br>$\;\;\;|$<br>$\;\;CH_3$ | 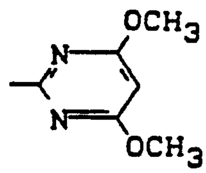 | 68 |
| (II-37) | $-OC_2H_5$ | 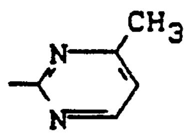 | 95 |
| (II-38) | $-OCH_3$ | 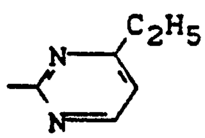 | 98 |
| (II-39) | $-OCH_3$ | 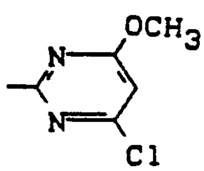 | 112 |
| (II-40) | $-OCH_3$ | 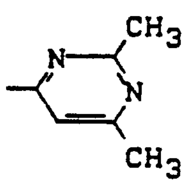 | 143 |
| (II-41) | $-OCH_3$ | 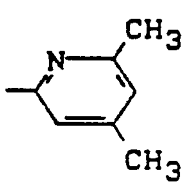 | 110 |

Tabelle 2 - Fortsetzung

| Beisp.-Nr. | $R^4$ | 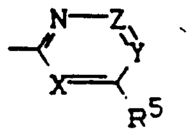 | Schmelzpunkt [°C] bzw. Brechungsindex |
|---|---|---|---|
| (II-42) | $-OCH_2-COOC_2H_5$ | 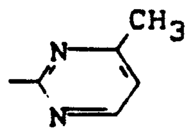 | |
| (II-43) | $-OCH_2-$ 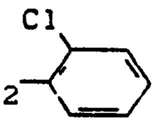 | 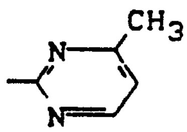 | 140 |
| (II-44) | $-OCH_2-$ 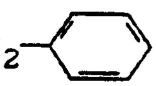 | 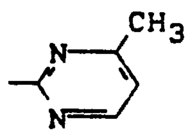 | 150 |
| (II-45) | $-OCH_2-$ 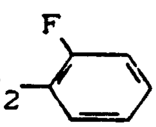 | 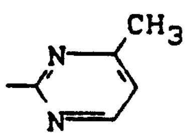 | 205 |
| (II-46) | $-OCH_2-CH=CH_2$ | 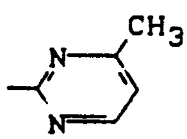 | |
| (II-47) | $-OC_4H_9(-n)$ | 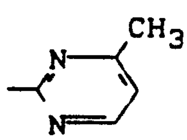 | |
| (II-48) | $-OC_4H_9(-s)$ | 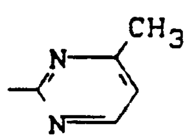 | |

Tabelle 2 - Fortsetzung

| Beisp.-Nr. | $R^4$ | 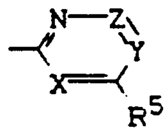 | Schmelzpunkt [°C] bzw. Brechungsindex |
|---|---|---|---|
| (II-49) | $-OCH_2CH_2CH_2Cl$ | 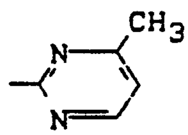 | 102 |
| (II-50) | $-OCH_3$ | 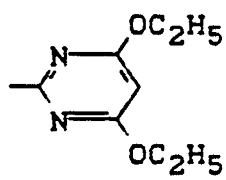 | |
| (II-51) | $-OCH_3$ | 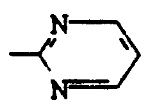 | 107 - 109 |
| (II-52) | $-OCH_2$ 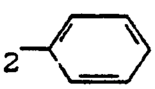 | 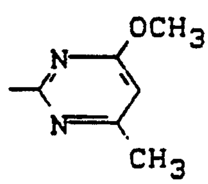 | $n_D^{20}$ = 1.5645 |
| (II-53) | $-OCH_2$ 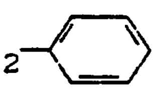 | 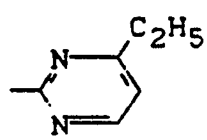 | 112 |
| (II-54) | $-OCH_2$ 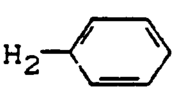 | 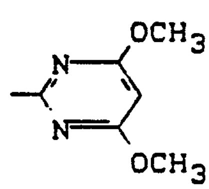 | 74 |

Tabelle 2 - Fortsetzung

| Beisp.-Nr. | $R^4$ | 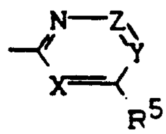 | Schmelzpunkt [°C] bzw. Brechungsindex |
|---|---|---|---|
| (II-55) | $-OCH_2$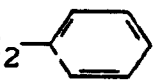 | 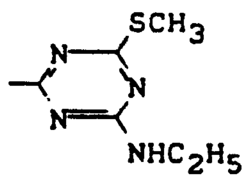 | 122 |
| (II-56) | $-OC_8H_{17}$(-n) | 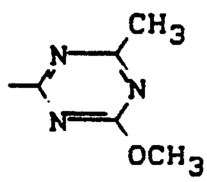 | 95 |
| (II-57) | $-OCH_2$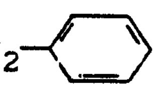 | 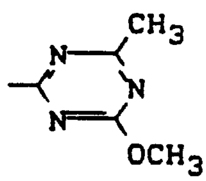 | 112 |
| (II-58) | $-OCH_3$ | 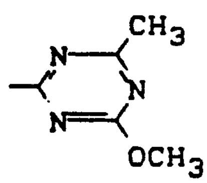 | 126 |
| (II-59) | $-OCH_2CH_2Cl$ | 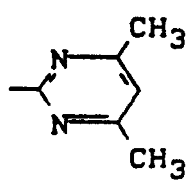 | (Öl) |
| (II-60) | $-OCH_2CH=CHCl$ | 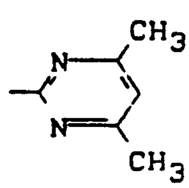 | 65 |

Tabelle 2 - Fortsetzung

| Beisp.-Nr. | $R^4$ | 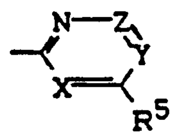 | Schmelzpunkt [°C] bzw. Brechungsindex |
|---|---|---|---|
| (II-61) | $-OCH_2CH_2CH_2CH_2Cl$ | 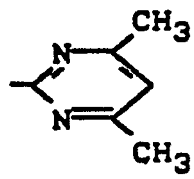 | Öl |
| (II-62) | $-OCH_2COOH$ | 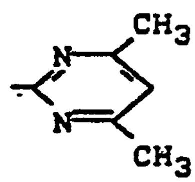 | 195 |
| (II-63) | -OCH 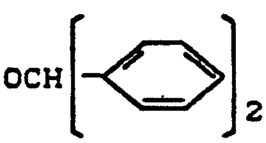 | 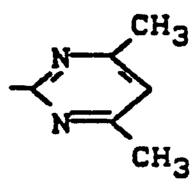 | 147 - 148 |
| (II-64) | $-OCH_2CH_2OCH_3$ | 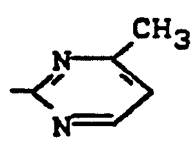 | 242 (Zers.) |
| (II-65) | -OCH 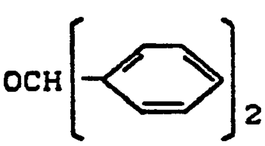 | 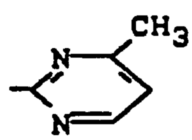 | 165 |
| (II-66) | $-OCH_2CONH_2$ | 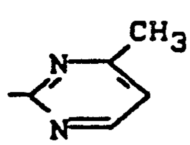 | 190 |

Tabelle 2 - Fortsetzung

| Beisp.-Nr. | $R^4$ | 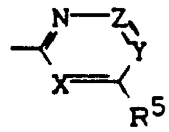 | Schmelzpunkt [°C] bzw. Brechungsindex |
|---|---|---|---|
| (II-67) | $-OCH_2CH=CH_2$ | 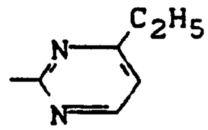 | 83 |
| (II-68) | $-OCH_3$ | 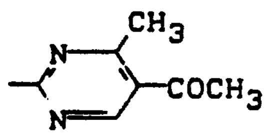 | 192 |
| (II-69) | $-OCH_3$ | 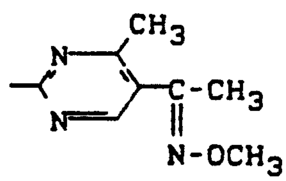 | 115 |
| (II-70) | $-OCH_2CH=CH_2$ | 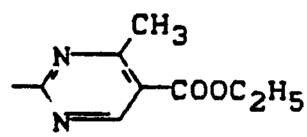 | 143 |
| (II-71) | $-OCH_2-$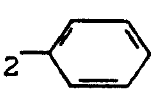 | 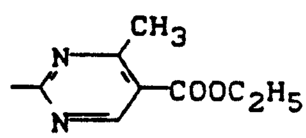 | 130 |
| (II-72) | $-OCH_3$ | 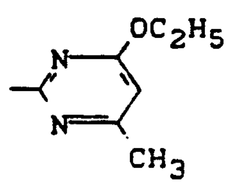 | $n_D^{20}$ = 1.5461 |
| (II-73) | $-OCH_3$ | 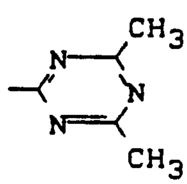 | 112 |

Herstellung von Ausgangsverbindungen der Formel (III)

Beispiel (III-1)

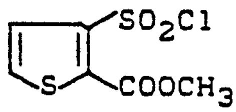

141,3 g (0,9 Mol) 3-Amino-2-methoxycarbonyl-thiophen werden in 568 ml 37 %iger Salzsäure 1 Stunde bei 25° C gerührt. Die erhaltene Suspension wird auf -3° C abgekühlt; bei dieser Temperatur wird unter Rühren eine Lösung von 62,7 g (0,91 Mol) Natriumnitrit in 112 ml Wasser zugetropft und noch 1 Stunde bei -3° C nachgerührt. Die so hergestellte Diazoniumsalzlösung tropft man in ein auf 5 -10° C abgekühltes Gemisch aus 472,0 g (7,38 Mol) Schwefeldioxid, 1050 ml Eisessig und 100 ml gesättigter wässriger Kupfer-(II)chlorid-Lösung. Man läßt unter Rühren auf 25 ° C erwärmen, rührt noch 2 Stunden bei 25 ° C nach und rührt das Reaktionsgemisch in 2 l Eiswasser ein. Man extrahiert 3 mal mit je 250 ml Methylenchlorid, wäscht die vereinigten Methylenchlorid-phasen mit Wasser, trocknet über Natiumsulfat, filtriert und engt unter vermindertem Druck ein.

Man erhält 173 g (80 % der Theorie) 2-Methoxycarbonyl-3-thienylsulfonsäurechlorid vom Schmelzpunkt 61-63° C.

Analog Beispiel (III-1) können die nachstehend aufgeführten Verbindungen der Formel (III) hergestellt werden:

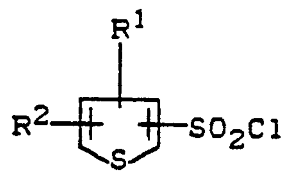

(III)

**Beispiel (III-2)**

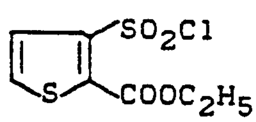

**Beispiel (III-3)**

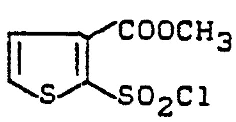

Herstellung von Ausgangsstoffen der Formel (VI)

Beispiel (VI-1)

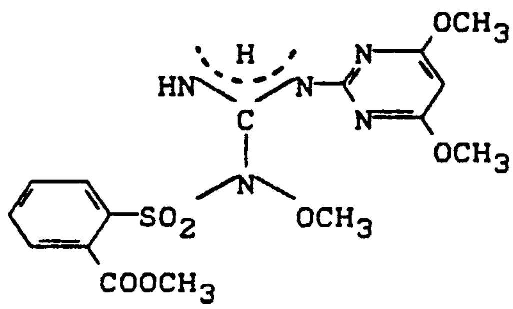

Eine Lösung aus 2,8 g (0,012 Mol) 2-Methoxycarbonyl-benzolsulfonsäurechlorid in 10 ml Tetrahydrofuran wird bei -5° C unter Rühren zu einer Mischung aus 2,3 g (0,01 Mol) N'-(4,6-Dimethoxy-pyrimidin-2-yl)-N"-methoxy-guanidin und 30 ml Tetrahydrofuran gegeben. Dann wird eine Lösung von 1,3 g (0,012 Mol) Diazabicyclooctan in 10 ml Tetrahydrofuran bei -5°C tropfenweise dazu gegeben und das Gemisch wird 2 Stunden bei 20° C gerührt. Nach Zugabe von weiteren 0,5 g 2-Methoxycarbonyl-benzolsulfonsäurechlorid und 0,25 g Diazabicylooctan wird weitere 2 Stunden bei 20°C gerührt. Nach Filtration wird eingeengt, der Rückstand mit Toluol zur Kristallisation gebracht und durch Absaugen isoliert.

Man erhält 2,25 g (57 % der Theorie) N'-(4,6-Dimethoxy-pyrimidin-2-yl)-N"-methoxy-N"-(2-methoxycarbonyl-benzolsulfonyl)-guanidin vom Schmelzpunkt 142-143° C. Analog können die in der nachstehenden Tabelle 3 aufgeführten Verbindungen der Formel (VI) hergestellt werden:

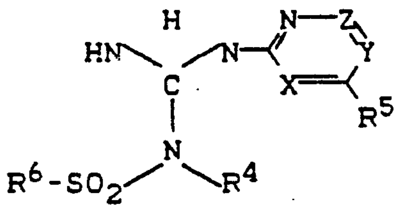

(VI)

Tabelle 3

| Beisp.-Nr. | $R^6$ | $R^4$ | 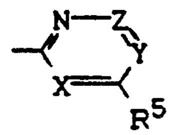 | Schmelzpunkt [°C] |
|---|---|---|---|---|
| (VI-2) | 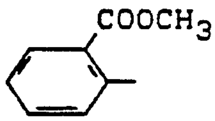 | $-OCH_3$ | 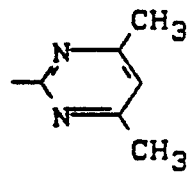 | 145 |
| (VI-3) | 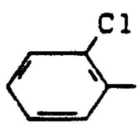 | $-OCH_3$ | 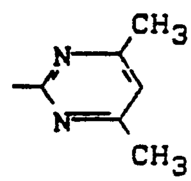 | 118 |
| (VI-4) | 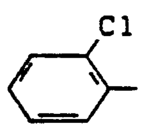 | $-OCH_3$ | 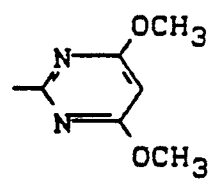 | (amorph) |
| (VI-5) | 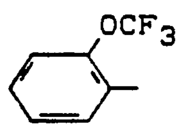 | $-OCH_3$ | 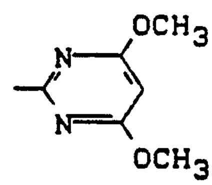 | (amorph) |

Tabelle 3 - Fortsetzung

| Beisp.-Nr. | $R^6$ | $R^4$ | 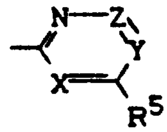 | Schmelzpunkt [°C] |
| --- | --- | --- | --- | --- |
| (VI-6) | 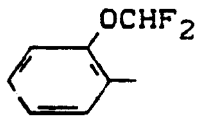 | $-OCH_3$ | 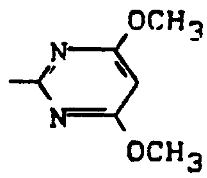 | (amorph) |
| (VI-7) | 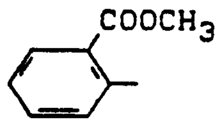 | $-OCH_3$ | 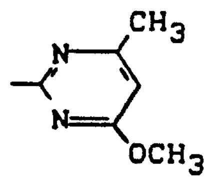 | |
| (VI-8) | 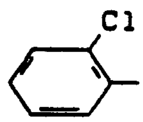 | $-OCH_3$ | 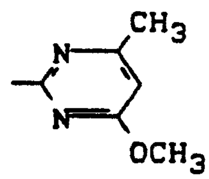 | |
| (VI-9) | 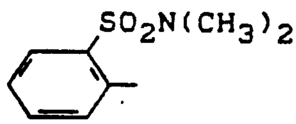 | $-OCH_3$ | 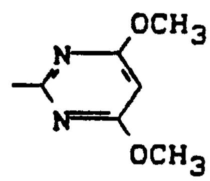 | |
| (VI-10) | 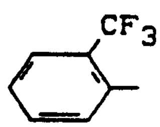 | $-OCH_3$ | 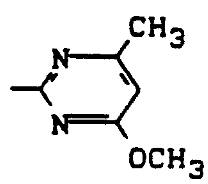 | |
| (VI-11) | 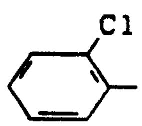 | $-OCH_3$ | 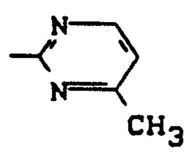 | 124 |

Tabelle 3 - Fortsetzung

| Beisp.-Nr. | $R^6$ | $R^4$ | 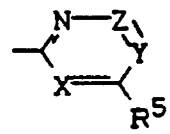 | Schmelzpunkt [°C] |
|---|---|---|---|---|
| (VI-12) | 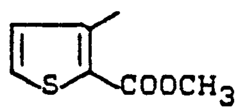 | $-OCH_3$ | 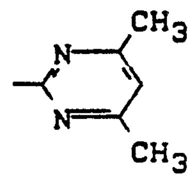 | 112 |
| (VI-13) | 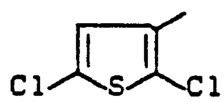 | $-OCH_3$ | 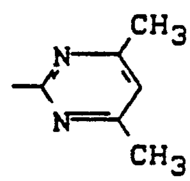 | amorph |
| (VI-14) | 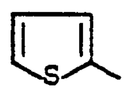 | $-OCH_3$ | 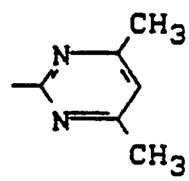 | 104 |
| (VI-15) | 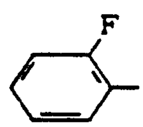 | $-OCH_3$ | 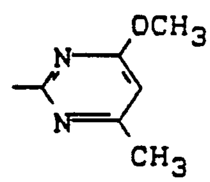 | 127 |
| (VI-16) | 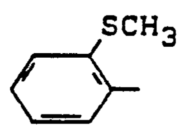 | $-OCH_3$ | 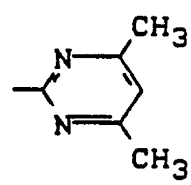 | |
| (VI-17) | 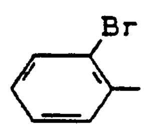 | $-OCH_3$ | 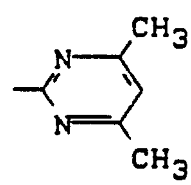 | |

Tabelle 3 - Fortsetzung

| Beisp.-Nr. | $R^6$ | $R^4$ | 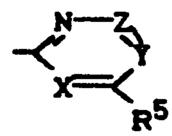 | Schmelzpunkt [°C] |
|---|---|---|---|---|
| (VI-18) | 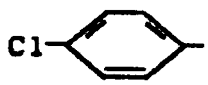 | $-OC_2H_5$ | 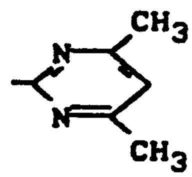 | |
| (VI-19) | 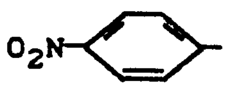 | $-OCH_2CH{=}CH_2$ | 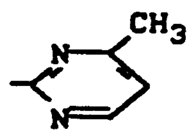 | |
| (VI-20) | 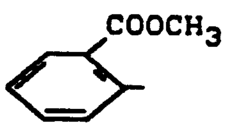 | $-OCH_3$ | 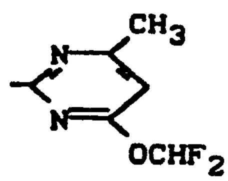 | |
| (VI-21) | $CH_3-$ | 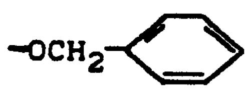 | 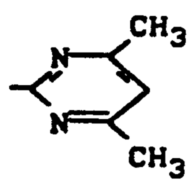 | |
| (VI-22) | $CH_3CH_2CH_2CH_2-$ | $-OCH_3$ | 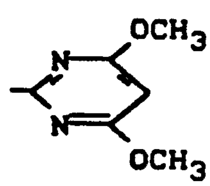 | |
| (VI-23) | $(CH_3)_2N-$ | $-OCH_3$ | 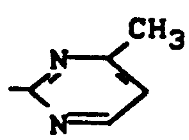 | |

Tabelle 3 - Fortsetzung

| Beisp.-Nr. | $R^6$ | $R^4$ | 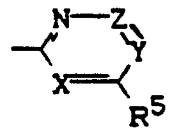 | Schmelzpunkt [°C] |
| --- | --- | --- | --- | --- |
| (VI-24) | $(n-)C_8F_{17}-$ | $-OCH_3$ | 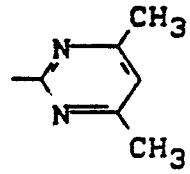 | |
| (VI-25) | 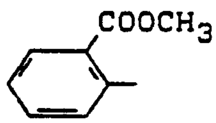 | $-OCH_3$ | 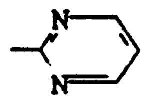 | 126 |
| (VI-26) | 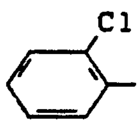 | $-OCH_3$ | 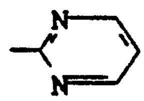 | 123 |
| (VI-27) | 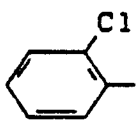 | 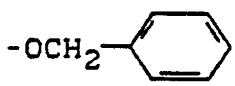 | 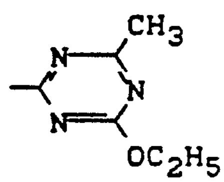 | (amorph) |
| (VI-28) | 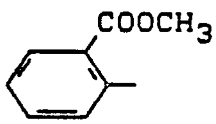 | 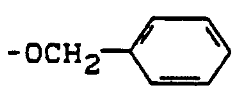 | 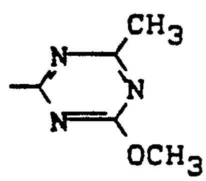 | (amorph) |
| (VI-29) | 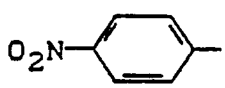 | $-OCH_3$ | 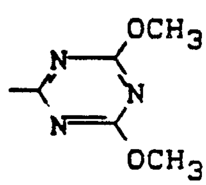 | |

Tabelle 3 - Fortsetzung

| Beisp.-Nr. | $R^6$ | $R^4$ | 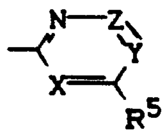 | Schmelzpunkt [°C] |
|---|---|---|---|---|
| (VI-30) | $CH_3$- | $-OCH_3$ | 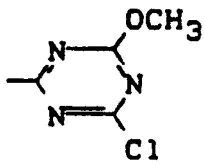 | |
| (VI-31) | 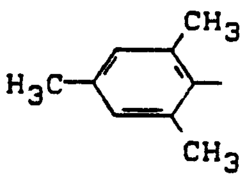 | $-OCH_3$ | 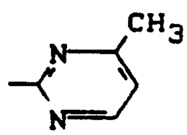 | 172 - 173 |
| (VI-32) | 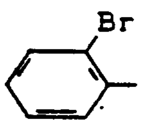 | $-OCH_3$ | 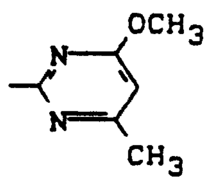 | 104 |
| (VI-33) | 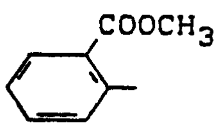 | $-OCH_3$ | 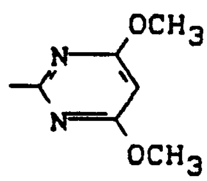 | 142 - 143 |
| (VI-34) | 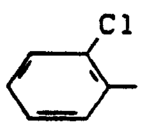 | 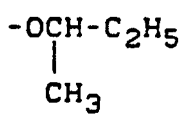 | 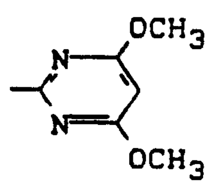 | 124 |
| (VI-35) | 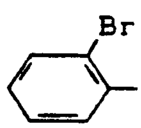 | $-OCH_3$ | 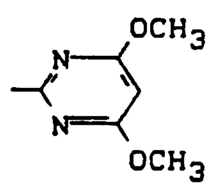 | (amorph) |

<u>Tabelle 3</u> - Fortsetzung

| Beisp.-Nr. | $R^6$ | $R^4$ | 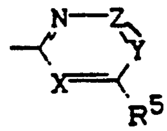 | Schmelzpunkt [°C] |
|---|---|---|---|---|
| (VI-36) | 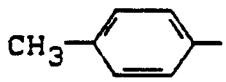 | $-OCH_3$ | 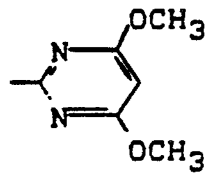 | (amorph) |
| (VI-37) | 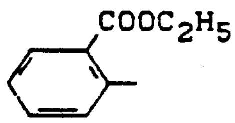 | $-OCH_3$ | 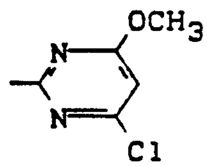 | 157 - 158 |
| (VI-38) | 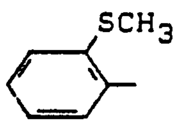 | $-OCH_3$ | 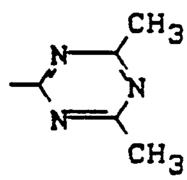 | 157 |
| (VI-39) | 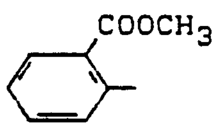 | $-OCH_3$ | 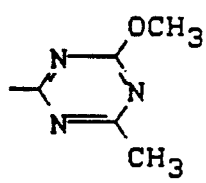 | 170 |
| (VI-40) | 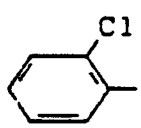 | $-OC_8H_{17}$ | 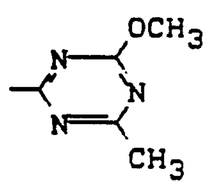 | 122 |
| (VI-41) | 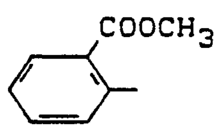 | $-OCH_3$ | 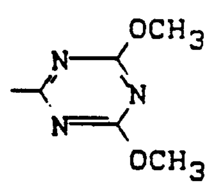 | 162 |

Tabelle 3 - Fortsetzung

| Beisp.-Nr. | $R^6$ | $R^4$ | 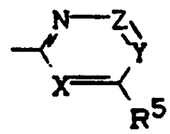 | Schmelzpunkt [°C] |
|---|---|---|---|---|
| (VI-42) | 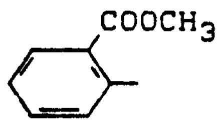 | $-OCH_3$ | 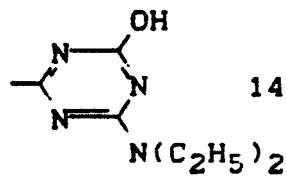 | 147 - 158 |
| (VI-43) | 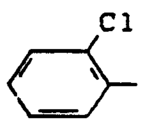 | $-OCH_3$ | 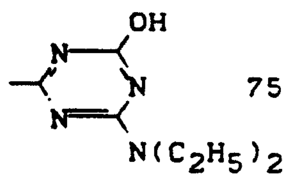 | 75 - 95 |
| (VI-44) | 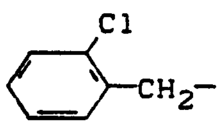 | $-OCH_3$ | 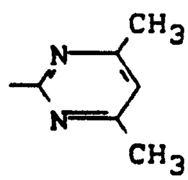 | 164 - 166 |
| (VI-45) | $CH_3-$ | $-OCH_3$ | 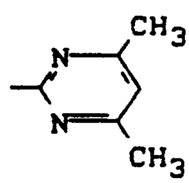 | 107 - 108 |
| (VI-46) | $CH_3-$ | $-OCH_2-CH(CH_3)_2$ | 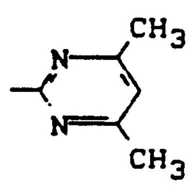 | 107 |
| (VI-47) | $CH_3-$ | $-OCH_2COOC_2H_5$ | 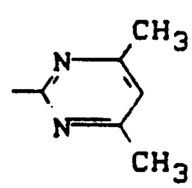 | 89 - 90 |

Tabelle 3 - Fortsetzung

| Beisp.-Nr. | $R^6$ | $R^4$ | 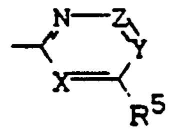 | Schmelzpunkt [°C] |
|---|---|---|---|---|
| (VI-48) | $(n-)C_4H_9-$ | $-OCH_3$ | 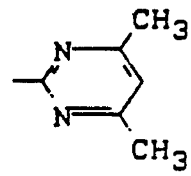 | 92 - 95 |
| (VI-49) | $(n-)C_4H_9-$ | $-OCH_2-$ 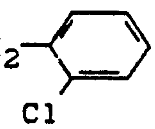 | 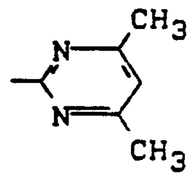 | 111 - 113 |
| (VI-50) | $Cl-CH_2CH_2CH_2CH_2-$ | $-OCH_3$ | 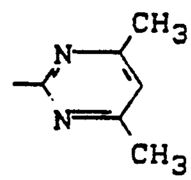 | 82 - 83 |
| (VI-51) | $C_8F_{17}-$ | $-OCH_3$ | 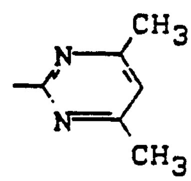 | 62 - 63 |
| (VI-52) | $(CH_3)_2N-$ | $-OCH_3$ | 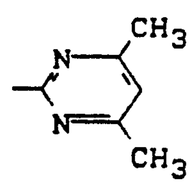 | 147 |
| (VI-53) | $(CH_3)_2N-$ | $-OCH_2COOC_2H_5$ | 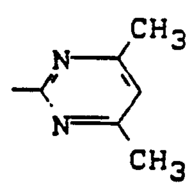 | 87 - 89 |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % -keine Wirkung (wie unbehandelte Kontrolle)

100 % -totale Vernichtung

Bei diesem Test zeigte z.B. die folgende Verbindung (1) der Herstellungsbeispiele eine ausgezeichnete Wirksamkeit gegen Unkräuter in z.B. Soja.

## Ansprüche

1) Thienylsulfonylguanidin-Derivate der allgemeinen Formel (I)

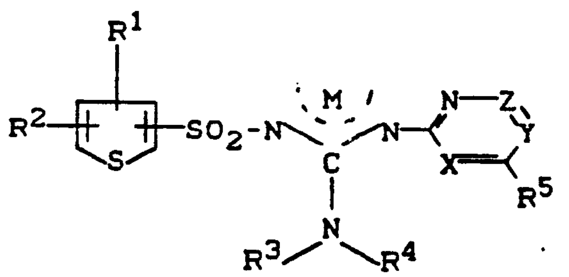

(I)

in welcher

$R^1$ und $R^2$ gleich oder verschieden sind und für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxycarbonyl [welches gegebenenfalls durch Fluor, Chlor und/oder $C_1$-$C_4$-Alkoxy substituiert ist] stehen,

$R^3$ für Wasserstoff, $C_1$-$C_4$-Alkyl oder für einen Sulfonylrest der Formel $R^6$-$SO_2$-steht, worin

$R^6$ für den Rest

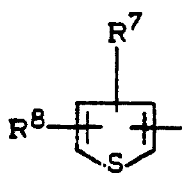

steht, worin

$R^7$ und $R^8$ gleich oder verschieden sind und für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-aminosulfonyl oder $C_1$-$C_4$-Alkoxycarbonyl [welches gegebenenfalls durch Fluor, Chlor und/oder $C_1$-$C_4$-Alkoxy substituiert ist] stehen,

in welcher weiter

$R^6$ für $C_1$-$C_{12}$-Alkyl [welches gegebenenfalls durch Fluor, Chlor, Brom, Cyano, Nitro, $C_1$-$C_4$-Alkoxy-carbonyl, $C_1$-$C_4$-Alkylamino-carbonyl, Di-($C_1$-$C_4$-alkyl)-amino-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_{12}$-Alkoxy [welches gegebenenfalls durch Fluor, Chlor, Brom, Cyano, Nitro, $C_1$-$C_4$-Alkoxycarbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_2$-$C_{12}$-Alkenyl [welches gegebenenfalls durch Fluor, Chlor, Brom, Cyano, Nitro, $C_1$-$C_4$Alkoxy, $C_1$-$C_4$-Alkylthio oder Phenyl substituiert ist], für $C_2$-$C_{12}$-Alkenyloxy [welches gegebenenfalls durch

Fluor, Chlor, Brom, Cyano, Nitro, $C_1$-$C_4$-Alkoxy, $C_1$-$C_4$-Alkylthio oder Phenyl substituiert ist], für $C_1$-$C_8$-Alkylamino, $C_3$-$C_6$-Cycloalkylamino, Di-($C_3$-$C_6$-cycloalkyl)-amino oder Di-($C_1$-$C_4$-alkyl)-amino steht [welche gegebenenfalls durch Fluor, Chlor, Brom, Cyano, Nitro, Phenyl, Phenoxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind], für $C_2$-$C_8$-Alkenylamino [welches gegebenenfalls durch Fluor, Chlor, Brom, Cyano, Nitro oder Phenyl substituiert ist], für Phenylamino oder Benzylamino - [welche gegebenenfalls durch Fluor, Chlor, Brom, $C_1$-$C_4$-Alkyl, Trifluormethyl, $C_1$-$C_4$-Alkoxy, $C_1$-$C_2$-Fluoralkoxy, $C_1$-$C_4$-Alkylthio, $C_1$-$C_4$-Alkylsulfinyl, $C_1$-$C_4$-Alkylsulfonyl, Cyano, Nitro und/oder $C_1$-$C_4$-Alkoxy-carbonyl substituiert sind], für Benzyl oder Phenyl steht, wobei letztere gegebenenfalls durch einen oder mehrere Reste aus der Reihe Halogen, Cyano, Nitro, $C_1$-$C_4$-Alkyl [welches gegebenenfalls durch Fluor, Chlor, Brom, Cyano, Carboxy, $C_1$-$C_4$-Alkoxy-carbonyl, $C_1$-$C_4$-Alkyl-amino-carbonyl, $C_1$-$C_4$-Alkoxy-amino-carbonyl, Di-($C_1$-$C_4$-alkyl)-amino-carbonyl, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkyl-amino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl oder Phenyl substituiert ist], $C_2$-$C_6$Alkenyl [welches gegebenenfalls durch Fluor, Chlor, Brom, Cyano, $C_1$-$C_4$-Alkoxy-carbonyl, Carboxy oder Phenyl substituiert ist], $C_2$-$C_6$-Alkinyl [welches gegebenenfalls durch Fluor, Chlor, Brom, Cyano, $C_1$-$C_4$-Alkoxy-carbonyl oder Phenyl substituiert ist], $C_1$-$C_4$-Alkoxy [welches gegebenenfalls durch Fluor, Chlor, Brom, Cyano, Carboxy, $C_1$-$C_4$-Alkylsulfinyl oder $C_1$-$C_4$-Alkylsulfonyl substituiert ist], $C_3$-$C_6$-Alkenoxy [welches gegebenenfalls durch Fluor, Chlor, Brom, Cyano oder $C_1$-$C_4$-Alkoxy-carbonyl substituiert ist], $C_3$-$C_6$-Alkenylthio [welches gegebenenfalls durch Fluor, Chlor, Brom, Cyano oder $C_1$-$C_4$-Alkoxy-carbonyl substituiert ist], $C_1$-$C_4$-Alkylthio, $C_1$-$C_4$-Alkylsulfinyl oder $C_1$-$C_4$-Alkylsulfonyl - [welche gegebenenfalls durch Fluor, Chlor, Brom, Cyano oder $C_1$-$C_4$-Alkoxy-carbonyl substituiert sind], Phenyl, Phenoxy, Di-($C_1$-$C_4$-alkyl)-amino-sulfonyl, $C_1$-$C_4$-Alkyl-amino-sulfonyl, $C_1$-$C_4$-Alkoxy-$C_1$-$C_4$-alkyl-amino-sulfonyl, $C_1$-$C_4$-Alkoxy-sulfonyl, $C_1$-$C_4$-Alkoxy-amino-sulfonyl, $C_1$-$C_6$-Alkoxy-carbonyl, $C_3$-$C_6$-Cycloalkoxy-carbonyl, $C_1$-$C_6$-Alkylamino-carbonyl, Di-($C_1$-C[ -alkyl)-amino-carbonyl, $C_1$-$C_6$-Alkoxy-amino-carbonyl, $C_1$-$C_4$-Alkoxy-$C_1$-$C_4$-alkyl-amino-carbonyl, $C_1$-$C_6$-Alkylaminosulfonylamino oder Di-($C_1$-$C_6$-alkyl)-amino-sulfonylamino substituiert sind und/oder gegebenenfalls benzanelliert sind;

in welcher weiter

$R^4$ für Wasserstoff, $C_1$-$C_6$-Alkyl [welches gegebenenfalls durch Fluor, Chlor, Brom, Cyano, Carboxy, $C_1$-$C_4$-Alkoxy-carbonyl, Hydroxy oder $C_1$-$C_4$-Alkoxy substituiert ist], $C_3$-$C_6$-Cycloalkyl, $C_3$-$C_6$-Alkenyl, $C_3$-$C_6$-Alkinyl, Phenylethyl oder Benzyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist] oder für Phenyl [welches gegebenenfalls durch Fluor, Chlor, Brom, Hydroxy, Cyano, Nitro, Amino, $C_1$-$C_4$-Alkyl, Trifluormethyl, $C_1$-$C_4$-Alkoxy, Trifluormethoxy, $C_1$-$C_4$-Alkylthio, Trifluormethylthio, Aminosulfonyl oder $C_1$-$C_4$-Alkoxy-carbonyl substituiert ist] steht, oder

$R^3$ und $R^4$ gemeinsam für $C_4$-$C_6$-Alkandiyl stehen [welches gegebenenfalls durch eine Sauerstoff-Brücke oder durch eine Brücke $>$N-$R^9$ unterbrochen ist], wobei

$R^9$ für $C_1$-$C_4$-Alkyl, $C_1$-$C_4$-Alkyl-carbonyl, $C_1$-$C_4$-Alkoxy-carbonyl oder Phenyl steht [welches gegebenenfalls durch Fluor, Chlor, Brom, Cyano, Nitro, $C_1$-$C_4$-Alkyl, $C_1$-$C_2$-Fluoralkyl oder $C_1$-$C_4$-Alkoxy substituiert ist];

in welcher weiter

$R^4$ für den Rest -$OR^{10}$ steht, worin

$R^{10}$ für $C_1$-$C_{12}$-Alkyl [ welches gegebenenfalls durch Fluor, Chlor, $C_1$-$C_4$-Alkoxy, $C_1$-$C_4$-Alkylthio, $C_1$-$C_4$-Alkylsulfinyl, $C_1$-$C_4$-Alkylsulfonyl, Carboxy, Cyano oder Nitro substituiert ist], $C_3$-$C_6$-Alkenyl [welches gegebenenfalls durch Fluor oder Chlor substituiert ist], $C_3$-$C_6$-Alkinyl, $C_3$-$C_6$-Cycloalkyl, $C_3$-$C_6$-Cycloalkyl-$C_1$-$C_2$-alkyl, $C_1$-$C_4$-Alkoxy-carbonyl-$C_1$-$C_2$-alkyl, Aminocarbonyl-$C_1$-$C_2$-alkyl, $C_1$-$C_4$-Alkyl-aminocarbonyl-$C_1$-$C_2$-alkyl, Di-($C_1$-$C_4$-alkyl)amino-carbonyl-$C_1$-$C_2$-alkyl, für Phenyl, Phenylethyl, Benzhydryl oder Benzyl [welche gegebenenfalls durch Fluor, Chlor, Brom, Cyano, Nitro, $C_1$-$C_4$-Alkyl, $C_1$-$C_4$-Alkoxy, Carboxy oder $C_1$-$C_4$-Alkoxy-carbonyl substituiert sind] steht und

in welcher weiter

$R^4$ für den Rest

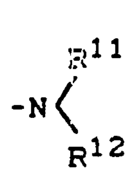

steht, worin

$R^{11}$ für Wasserstoff oder $C_1$-$C_4$-Alkyl steht und

$R^{12}$ für Wasserstoff, $C_1$-$C_4$-Alkyl [welches gegebenenfalls durch Halogen, Cyano, Nitro, $C_1$-$C_4$-Alkoxy oder $C_1$-

$C_4$-Alkoxy-carbonyl substituiert ist], $C_3$-$C_6$-Cycloalkyl, Phenyl-$C_1$-$C_2$-alkyl, Phenyl [welche gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio oder $C_1$-$C_4$-Alkoxy-carbonyl substituiert sind], für $C_1$-$C_4$-Alkyl-carbonyl, $C_1$-$C_4$-Alkoxy-carbonyl, $C_1$-$C_4$-Alkylsulfonyl, $C_1$-$C_4$-Halogenalkylsulfonyl oder Phenylsulfonyl [welches gegebenenfalls durch Halogen, Cyano, Nitro, $C_1$-$C_4$-Alkyl, $C_1$-$C_4$-Halogenalkyl, $C_1$-$C_4$-Alkoxy, $C_1$-$C_2$-Halogenalkoxy oder $C_1$-$C_4$-Alkoxy-carbonyl substituiert ist] steht,

$R^5$ für Wasserstoff, Halogen, Hydroxy, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino oder Di-($C_1$-$C_4$-alkyl)-amino steht,

X für Stickstoff oder eine -CH-Gruppierung steht,

Y für Stickstoff oder eine -$CR^{13}$-Gruppierung steht, worin

$R^{13}$ für Wasserstoff, Halogen, $C_1$-$C_4$-Alkyl, Formyl, $C_1$-$C_4$-Alkyl-carbonyl, $C_1$-$C_4$-Alkoxy-carbonyl oder $C_1$-$C_2$-Alkoximino-($C_1$-$C_2$-alkyl) steht, und

Z für Stickstoff oder eine -$CR^{14}$-Guppierung steht, worin

$R^{14}$ für Wasserstoff, Halogen, Hydroxy, $C_1$-$C_4$-Alkyl, $C_1$-$C_4$-Alkoxy, $C_1$-$C_4$-Alkylthio, $C_1$-$C_4$-Alkylamino oder Di-($C_1$-$C_4$-alkyl)amino steht und

M für Wasserstoff oder ein Metalläquivalent steht,

sowie 1:1-Addukte von Verbindungen der Formel (I) mit starken Säuren.

2) Verfahren zur Herstellung von Thienylsulfonylguanidin-Derivaten der allgemeinen Formel (I), gemäß Anspruch 1, dadurch gekennzeichnet, daß man

(a) für den Fall, daß M für Wasserstoff steht,

Guanidin-Derivate der Formel (II)

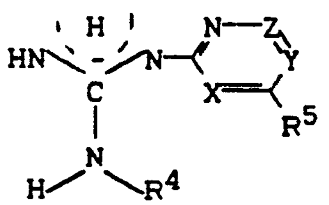

(II)

in welcher

$R^4$, $R^5$, X, Y und Z die oben angegebenen Bedeutungen haben,

mit Sulfonsäurechloriden der Formel (III)

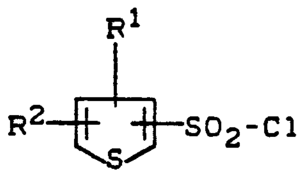

(III)

in welcher

$R^1$ und $R^2$ die oben angegebenen Bedeutungen haben,

in Gegenwart von Säureakzeptoren und gegebenenfalls in Gegenwart von Verdünnungsmitteln umsetzt; oder daß man

(b) für den Fall, daß M für Wasserstoff steht und $R^3$ für Wasserstoff oder $C_1$-$C_4$-Alkyl steht,

die nach dem oben unter (a) angegebenen Verfahren erhältlichen Thienylsulfonylguanidin-Derivate der Formel (ID)

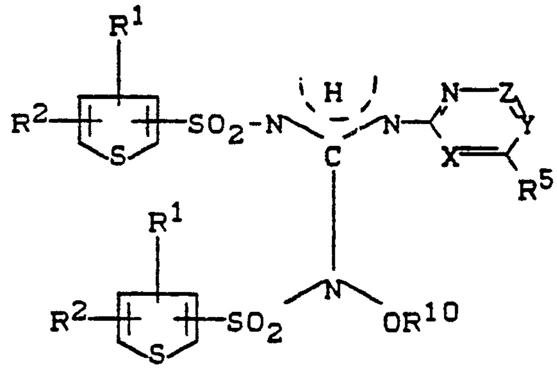

(ID)

in welcher
$R^1$, $R^2$, $R^5$, $R^{10}$, X, Y und Z die oben angegebenen Bedeutungen haben,
mit Aminoverbindungen der Formel (IV)

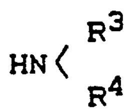

(IV)

in welcher
$R^3$ für Wasserstoff oder $C_1$-$C_4$-Alkyl steht und
$R^4$ die oben angegebene Bedeutung hat,
bzw. mit Hydrochloriden von Aminoverbindungen der Formel (IV), gegebenenfalls in Gegenwart von Säureakzeptoren und gegebenenfalls in Gegenwart von Verdünnungsmitteln umsetzt;
oder daß man

(c) für den Fall, daß M für Wasserstoff steht und
$R^3$ für einen Sulfonylrest der Formel $R^6$-$SO_2$-steht, worin
$R^6$ die oben angegebene Bedeutung hat,
Thienylsulfonylguanidin-Derivate der Formel (IE)

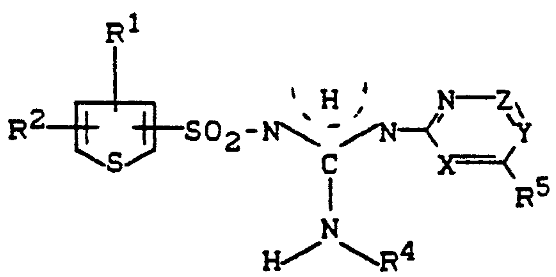

(IE)

in welcher
$R^1$, $R^2$, $R^4$, $R^5$, X, Y und Z die oben angegebenen Bedeutungen haben,
mit Sulfonsäurechloriden der Formel (V)
$R^6$-$SO_2$-Cl (V)
in welcher
$R^6$ die oben angegebene Bedeutung hat,
in Gegenwart von Säureakzeptoren und gegebenenfalls in Gegenwart von Verdünnungsmitteln umsetzt;
oder daß man

(d) ebenfalls für den Fall, daß M für Wasserstoff steht und
$R^3$ für einen Sulfonylrest der Formel $R^6$-$SO_2$-steht, worin
$R^6$ die oben angegebene Bedeutung hat,
Sulfonylguanidin-Derivate der Formel (VI)

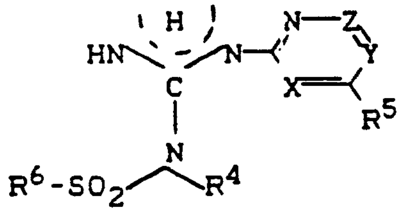

(VI)

in welcher
$R^4$, $R^5$, $R^6$, X, Y und Z die oben angegebenen Bedeutungen haben,
mit Sulfonsäurechloriden der Formel (III)

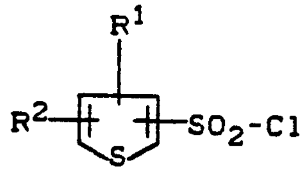

(III)

in welcher
$R^1$ und $R^2$ die oben angegebenen Bedeutungen haben,
in Gegenwart von Säureakzeptoren und gegebenenfalls in Gegenwart von Verdünnungsmitteln umsetzt;
oder daß man

(e) für den Fall, daß M für Wasserstoff steht und
$R^3$ für Wasserstoff oder $C_1$-$C_4$-Alkyl steht,
Sulfonylisothioharnstoff-Derivate der Formel (VII)

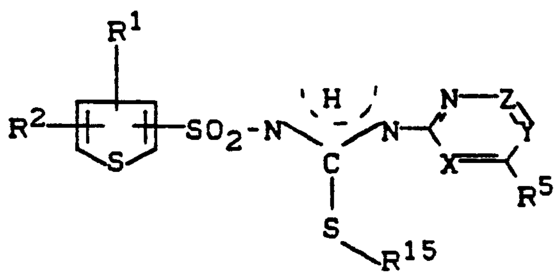

(VII)

in welcher
$R^1$, $R^2$, $R^5$, X, Y und Z die oben angegebenen Bedeutungen haben und
$R^{15}$ für $C_1$-$C_4$-Alkyl oder Benzyl steht,
mit Aminoverbindungen der Formel (IV)

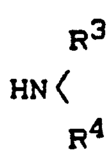

(IV)

in welcher
$R^3$ für Wasserstoff oder $C_1$-$C_4$-Alkyl steht und
$R^4$ die oben angegebene Bedeutung hat,
gegebenenfalls in Gegenwart von Verdünnungsmitteln umsetzt;
oder daß man

(f) für den Fall, daß M für ein Metalläquivalent steht, die nach den oben unter (a), (b), (c), (d) und (e) angegebenen Verfahren erhältlichen Verbindungen der Formel (I), in welcher M für Wasserstoff steht und $R^1$, $R^2$, $R^3$, $R^4$, $R^5$, X, Y und Z die oben angegebenen Bedeutungen haben,

mit Metallhydroxiden, -hydriden oder -alkanolaten oder mit metallorganischen Verbindungen, gegebenenfalls in Gegenwart von Verdünnungsmitteln umsetzt;
oder daß man

(g) für den Fall, daß 1:1-Addukte von Verbindungen der Formel (I) mit starken Säuren herzustellen sind,
Verbindungen der Formel (I), in welcher M für Wasserstoff steht und $R^1$, $R^2$, $R^3$, $R^4$, $R^5$, X, Y und Z die oben angegebenen Bedeutungen haben,
mit starken Säuren, gegebenenfalls in Gegenwart von Verdünnungsmitteln umsetzt.

3) Herbizide Mittel, gekennzeichnet durch einen Gehalt an mindestens einem Thienylsulfonylguanidin-Derivat der allgemeinen Formel (I) gemäß Anspruch 1.

4) Verwendung von Thienylsulfonylguanidin-Derivaten der allgemeinen Formel (I) gemäß Anspruch 1 zur Bekämpfung von unerwünschtem Pflanzenwachstum.

5) Verfahren zur Herstellung von herbiziden Mitteln, dadurch gekennzeichnet, daß man Thienylsulfonylguanidin-Derivate der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 86115204.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 121 082 (BAYER)<br>* Formel I und Seite 15, Zeilen 17ff; Ansprüche 4-7 *<br>-- | 1,3-5 | C 07 D 409/12<br>A 01 N 47/44 |
| A | EP - A2 - 0 124 295 (DU PONT)<br>* Formel I *<br>-- | 3-5 | |
| P,A | EP - A1 - 0 173 323 (BAYER)<br>* Ansprüche 1,3 *<br>-- | 1,3-5 | |
| P,A | EP - A1 - 0 173 956 (BAYER)<br>* Ansprüche 1,3,4,5 *<br>-- | 1,3-5 | |
| A | EP - A2 - 0 064 804 (DU PONT)<br>* Tabelle IIIB; Anspruch 1 *<br>---- | 1 | |

RECHERCHIERTE SACHGEBIETE (Int. Cl.4)

C 07 D 409/00

Der vorliegende Recherchenbericht wurde für alle Patentansprüche erstellt.

| Recherchenort | Abschlußdatum der Recherche | Prüfer |
|---|---|---|
| WIEN | 13-02-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument